# EUROPÄISCHE PATENTSCHRIFT

(11) **EP 1 554 278 B1**
(45) Veröffentlichungstag und Bekanntmachung des Hinweises auf die Patenterteilung: **12.04.2006**
(21) Anmeldenummer: 03792342.2
(22) Anmeldetag: 16.08.2003
(51) Int. Cl.: C07D 473/00

(54) **NEUE XANTHINDERIVATE, DEREN HERSTELLUNG UND DEREN VERWENDUNG ALS ARZNEIMITTEL**
XANTHINE DERIVATIVES, PRODUCTION THEREOF AND USE THEREOF AS MEDICINES
DERIVES DE XANTHINE, LEUR PRODUCTION ET LEUR UTILISATION COMME MEDICAMENTS

(30) Priorität: 22.08.2002 DE 10238470
(43) Veröffentlichungstag der Anmeldung: 20.07.2005
(73) Patentinhaber: Boehringer Ingelheim Pharma GmbH & Co. KG, 55216 Ingelheim am Rhein (DE)
(72) Erfinder: ECKHARDT, Matthias, 88400 Biberach an der Riss (DE); MARK, Michael, 88400 Biberach an der Riss (DE); HIMMELSBACH, Frank, 88441 Mittelbiberach (DE); LANGKOPF, Elke, 88447 Warthausen (DE); LOTZ, Ralf, 88433 Schemmerhofen (DE); MAIER, Roland, 88400 Biberach an der Riss (DE)
(86) Internationale Anmeldenummer: PCT/EP2003/009096
(87) Internationale Veröffentlichungsnummer: WO 2004/018467

(56) Entgegenhaltungen:
- EP-A- 1 338 595
- WO-A-02/02560
- WO-A-02/068420
- WO-A-03/004496
- WO-A-03/024965
- WO-A-03/057200

## Beschreibung

Gegenstand der vorliegenden Erfindung sind neue substituierte Xanthine der allgemeinen Formel 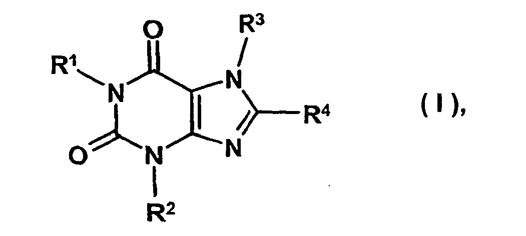 deren Tautomere, Enantiomere, Diastereomere, deren Gemische, deren Prodrugs und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

In der obigen Formel 1 bedeuten
R¹ eine Phenylcarbonylmethylgruppe, in der der Phenylteil durch R¹⁰ und R¹¹ substituiert ist, wobei
R¹⁰ eine Formylaminogruppe,
eine C₃₋₇-Cycloalkyl-carbonylamino- oder C₃₋₇-Cycloalkyl-C₁₋₃-alkyl-carbonylamino-Gruppe,
eine C₆₋₉-Bicycloalkyl-carbonylamino- oder C₆₋₉-Bicycloalkyl-C₁₋₃-alkyl-carbonylaminogruppe,
eine C₅₋₇-Cycloalkyl-carbonylaminogruppe, in der
eine Methylengruppe durch ein Sauerstoff- oder Schwefelatom oder durch eine Imino-, Sulfinyl- oder Sulfonylgruppe ersetzt ist,
eine C₅₋₇-Cycloalkyl-carbonylaminogruppe, in der eine -CH₂-CH₂- Gruppe durch eine -NH-CO- oder-NH-NH- Gruppe ersetzt ist,
eine C₅₋₇-Cycloalkyl-carbonylaminogruppe, in der eine -CH₂-CH₂CH₂- Gruppe durch eine -NH-CO-NH-, -NH-CO-O- oder-O-CH₂-O- Gruppe ersetzt ist,
eine C₆₋₇-Cycloalkyl-carbonylaminogruppe, in der eine -CH₂-CH₂-CH₂-CH₂₋Gruppe durch eine -NH-CH₂-CH₂-NH-, -NH-CO-CH₂-NH-, -NH-CH₂-CH₂-O-, -NH-CO-CH₂-O- oder -O-CH₂-CH₂-O-Gruppe ersetzt ist,
eine Cycloheptyl-carbonylaminogruppe, in der eine -CH₂-CH₂-CH₂-CH₂-CH₂₋Gruppe durch eine -NH-CH₂CH₂-CH₂-NH-, -NH-CO-CH₂-CH₂-NH-, -NH-CH₂-CH₂-CH₂-O-, -NH-CO-CH₂-CH₂-O- oder -O-CH₂-CH₂-CH₂-O-Gruppe ersetzt ist,
eine C₅₋₇-Cycloalkyl-carbonylaminogruppe, in der eine oder zwei Methylengruppen durch Carbonylgruppen ersetzt sind,
eine C₄₋₇-Cycloalkenyl-carbonylamino- oder C₄₋₇-Cycloalkenyl-C₁₋₃-alkyl-carbonylamino-Gruppe,
eine C₃₋₇-Cycloalkyl-sulfonylamino-, C₃₋₇-Cycloalkyl-C₁₋₃-alkyl-sulfonylamino-, Arylsulfonylamino- oder Aryl-C₁₋₃-alkyl-sulfonylamino-Gruppe oder
eine Heteroarylcarbonylaminogruppe bedeutet,
wobei die in den vorstehend erwähnten Gruppen enthaltenen Iminogruppen unabhängig voneinander durch eine C₁₋₃-Alkylgruppe substituiert sein können,
und R¹¹ ein Wasserstoff-, Fluor-, Chlor-, Brom- oder lodatom oder
eine C₁₋₃-Alkyl-, C₁₋₃-Alkyloxy-, Difluormethyl-, Trifluormethyl-, Difluormethoxy-, Trifluormethoxy- oder Cyangruppe bedeutet,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Arylgruppe,
eine Aryl-C₁₋₄-alkylgruppe,
eine Aryl-C₂₋₃-alkenylgruppe,
eine Arylcarbonyl-C₁₋₂-alkylgruppe,
eine Heteroaryl-C₁₋₃-alkylgruppe,
eine Furanylcarbonylmethyl-, Thienylcarbonylmethyl-, Thiazolylcarbonylmethyl- oder Pyridylcarbonylmethylgruppe,
eine C₁₋₄Alkyl-carbonyl-C₁₋₂-alkyl-Gruppe,
eine C₃₋₆-Cycloalkyl-carbonyl-C₁₋₂-alkyl-Gruppe,
eine Aryl-D-C₁₋₃-alkylgruppe, wobei D eine Sauerstoff- oder Schwefelatom, eine Imino-, C₁₋₃-Alkylimino-, Sulfinyl- oder Sulfonylgruppe bedeutet,
eine durch eine Gruppe Rₐ substituierte C₁₋₄-Alkylgruppe, wobei
Rₐ eine Cyano-, Carboxy-, C₁₋₃-Alkyloxy-carbonyl-, Aminocarbonyl-, C₁₋₃₋Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet,
oder eine durch eine Gruppe R_{b} substituierte C₂₋₄-Alkylgruppe, wobei
R_{b} eine Hydroxy-, C₁₋₃-Alkyloxy-, Amino-, C₁₋₃-Alkylamino-, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1-yl-Gruppe darstellt und durch mindestens zwei Kohlenstoffatome vom Ringstickstoffatom in 3-Stellung des Xanthingerüstes isoliert ist,
R³ eine C₃₋₈-Alkylgruppe,
eine durch eine Gruppe R_{c} substituierte C₁₋₃-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇₋Cycloalkenylgruppe,
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom, oder eine Trifluorrnethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1 yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-lmino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃₋alkyl)-aminogruppe substituiert ist,
eine C₃₋₇-Cycloalkylgruppe, die durch eine Amino-C₁₋₃-alkyl-, C₁₋₃-Alkylamino-C₁₋₃₋alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃₋alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃₋Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃₋alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃₋alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃₋alkylgruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino-Teils getrennt ist, wobei R¹⁹ wie vorstehend erwähnt definiert ist,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃₋Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-; Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2*H-*benzo[1,4]oxazinyl-Gruppe zu verstehen ist,
und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische, deren Prodrugs und deren Salze.

Verbindungen, die eine in-vivo abspaltbare Gruppe enthalten, sind Prodrugs der entsprechenden Verbindungen, bei denen diese in-vivo abspaltbare Gruppe abgespalten ist.

Die bei der Definition der vorstehend erwähnten Reste erwähnten Carboxygruppen können durch eine in-vivo in eine Carboxygruppe überführbare Gruppe oder durch eine unter physiologischen Bedingungen negativ geladene Gruppe ersetzt sein,
desweiteren können die bei der Definition der vorstehend erwähnten Reste erwähnten Amino- und Iminogruppen durch einen in-vivo abspaltbaren Rest substituiert sein. Derartige Gruppen werden beispielsweise in der WO 98/46576 und von N.M. Nielsen et al. in International Joumal of Pharmaceutics 39, 75-85 (1987) beschrieben.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkyloxycarbonyl- oder C₂₋₆-Alkanoylgruppe substituierte lminogruppe ersetzt ist und der Cycloalkanolteil zusätzlich durch ein oder zwei C₁-₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel

Rₚ-CO-O-(R_{q}CRᵣ)-OH,

in dem
Rₚ eine C₁₋₈-Alkyl-, C₅₋₇-Cycloalkyl-, C₁₋₈-Alkyloxy-, C₅₋₇-Cycloalkyloxy-, Phenyl- oder Phenyl- C₁₋₃-alkylgruppe,
R_{q} ein Wasserstoffatom, eine C₁₋₃-Alkyl-, C₅₋₇-Cycloalkyl- oder Phenylgruppe und
Rᵣ ein Wasserstoffatom oder eine C₁₋₃-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylaminocarbonyl-, Benzylsulfonylaminocarbonyl- oder Perfluor-C₁₋₆-alkylsulfonylaminocarbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkyloxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁₋₁₆-Alkyloxycarbonyl- oder C₁₋₁₆-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-C₁₋₆-alkyloxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkyloxycarbonyl-, C₁₋₃-Alkyloxy-C₂₋₄-alkyloxy-C₂₋₄-alkyloxycarbonyl-, Rₚ-CO-O-(R_{q}CRᵣ)-O-CO-, C₁₋₆-Alkyl-CO-N H-(RₛCRₜ)-O-CO- oder C₁₋₆-Alkyl-CO-O-(RₛCRₜ)-(RₛCRₜ)-O-CO-Gruppe, in denen Rₚ bis Rᵣ wie vorstehend erwähnt definiert sind,
Rₛ und Rₜ, die gleich oder verschieden sein können, Wasserstoffatome oder C₁₋₃-Alkylgruppen darstellen,
zu verstehen.

Desweiteren schließen die in den vor- und nachstehenden Definitionen erwähnten gesättigten Alkyl- und Alkyloxyteile, die mehr als 2 Kohlenstoffatome enthalten, soweit nichts Anderes erwähnt wurde, auch deren verzweigte Isomere wie beispielsweise die Isopropyl-, tert.Butyl-, lsobutylgruppe etc. ein.

Bevorzugt sind diejenigen Verbindungen der allgemeinen Formel I, in denen
R¹, R² und R³ wie oben erwähnt definiert sind und
R⁴ eine Pyrrolidin-1-ylgruppe, die in 3-Stellung durch eine Aminogruppe substituiert ist,
eine Piperidin-1-ylgruppe, die in 3-Stellung durch eine Aminogruppe substituiert ist, eine Hexahydroazepin-1-yl-gruppe, die in 3-Stellung oder in 4-Stellung durch eine Aminogruppe substituiert ist,
eine (2-Aminocyclohexyl)amino-Gruppe,
eine Cyclohexylgruppe, die in 3-Stellung durch eine Aminogruppe substituiert ist, oder
eine N-(2-Aminoethyl)-methylamino- oder eine N-(2-Aminoethyl)-ethylamino-Gruppe bedeutet,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische und Salze.

Besonders bevorzugt sind diejenigen Verbindungen der allgemeinen Formel I, in denen
R¹ eine Phenylcarbonylmethylgruppe, in der der Phenylteil durch R¹⁰ substituiert ist, wobei
R¹⁰ eine Formylaminogruppe,
eine C₃₋₇-Cycloalkyl-carbonylamino- oder C₃₋₇-Cycloalkyl-C₁₋₃-alkyl-carbonylamino-Gruppe,
eine C₆₋₉-Bicycloalkyl-carbonylaminogruppe,
eine C₅₋₇-Cycloalkyl-carbonylaminogruppe, in der
eine Methylengruppe durch ein Sauerstoff- oder Schwefelatom oder durch eine Imino-, Sulfinyl- oder Sulfonylgruppe ersetzt ist,
eine (1,3-Dioxolanyl)-carbonylamino-, (1,4-Dioxanyl)-carbonylamino-, Morpholin-2-yl-carbonylamino-, Morpholin-3-ylcarbonylamino- oder Piperazin-2-yl-carbonylamino-Gruppe,
eine C₅₋₇-Cycloalkyl-carbonylaminogruppe, in der eine -CH₂-CH₂- Gruppe durch eine -NH-CO- Gruppe ersetzt ist,
eine C₅₋₇-Cycloakyl-carbonylaminogruppe, in der eine -CH₂-CH₂-CH₂- Gruppe durch eine -NH-CO-O- Gruppe ersetzt ist,
eine C₅₋₇-Cycloalkyl-carbonylaminogruppe, in der eine Methylengruppe durch eine Carbonylgruppe ersetzt ist,
eine C₅₋₇-Cycloalkenyl-carbonylamino- oder C₅₋₇-Cycloalkenyl-C₁₋₃-alkyl-carbonylamino-Gruppe,
eine C₃₋₇-Cycloalkyl-sulfonylamino-, Phenylsulfonylamino- oder Phenyl-C₁₋₃₋alkyl-sulfonylamino-Gruppe oder
eine Pyridinylcarbonylaminogruppe bedeutet,
R² ein Wasserstoffatom,
oder eine C₁₋₃-Alkylgruppe,
R³ eine C₄₋₆-Alkenylgruppe,
eine 2-Butin-1-ylgruppe oder
eine 1-Cyclopenten-1-yl-methyl-Gruppe
und
R⁴ eine Piperidin-1-ylgruppe, die in 3-Stellung durch eine Aminogruppe substituiert ist,
eine Hexahydroazepin-1-yl-gruppe, die in 3-Stellung oder in 4-Stellung durch eine Aminogruppe substituiert ist,
eine (2-Aminocyclohexyl)amino-Gruppe,
eine Cyclohexylgruppe, die in 3-Stellung durch eine Aminogruppe substituiert ist, oder
eine N-(2-Aminoethyl)-methylamino- oder eine N-(2-Aminoethyl)-ethylamino-Gruppe bedeuten,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische und deren Salze.

Ganz besonders bevorzugt sind diejenigen Verbindungen der allgemeinen Formel I, in denen
R¹ eine Phenylcarbonylmethyl-Gruppe, in der der Phenylteil durch eine Formylamino, Pyridinylcarbonylamino- oder Cyclopropylcarbonylamino-Gruppe substituiert ist,
R² eine Methylgruppe,
R³ eine 2-Buten-1-yl- oder 3-Methyl-2-buten-1-yl-Gruppe oder
eine 2-Butin-1-yl-Gruppe
und
R⁴ eine (3-Amino-piperidin-1-yl)-Gruppe bedeuten,
deren Tautomere, Enantiomere, Diastereomere, deren Gemische und deren Salze,
insbesondere jedoch diejenigen Verbindungen der allgemeinen Formel I, in denen
R¹ eine [2-(Cyclopropylcarbonylamino)-phenyl]-carbonylmethyl- oder [2-(Pyridyl-carbonylamino)-phenyl]-carbonylmethyl-Gruppe,
R² eine Methylgruppe,
R³ eine 2-Buten-1-yl- oder 3-Methyl-2-buten-1-yl-Gruppe oder
eine 2-Butin-1-yl-Gruppe
und
R⁴ eine (3-Amino-piperidin-1-yl)-Gruppe bedeuten,
deren Tautomere, Enantiomere, Diastereomere, deren Gemische und deren Salze.

Folgende Verbindungen der allgemeinen Formel I sind besonders bevorzugt:
(1) 1-[2-(2-Formylamino-phenyl)-2-oxo-ethyl]-3-methyl-7-(3-methyl-2-buten-1-yl)-8-(3-amino-piperidin-1-yl)-xanthin,
(2) 1-(2-{2-[(Cyclopropylcarbonyl)amino]-phenyl}-2-oxo-ethyl)-3-methyl-7-(3-methyl-2-buten-1-yl)-8-(3-amino-piperidin-1-yl)-xanthin,
(3) 1-[2-(2-Formylamino-phenyl)-2-oxo-ethyl]-3-methyl-7-(2-butin-1-yl)-8-(3-amino-piperidin-1-yl)-xanthin,
(4) 1-(2-{2-[(Cyclopropylcarbonyl)amino]-phenyl}-2-oxo-ethyl)-3-methyl-7-((E)-2-buten-1-yl)-8-((*R*)-3-amino-piperidin-1-yl)-xanthin,
(5) 1-(2-{2-[(Cyclopropylcarbonyl)amino]-phenyl}-2-oxo-ethyl)-3-methyl-7-((E)-2-buten-1-yl)-8-((*S*)-3-amino-piperidin-1-yl)-xanthin,
(6) 1-(2-{2-[(Cyclopropylcarbonyl)amino]-phenyl}-2-oxo-ethyl)-3-methyl-7-(2-butin-1-yl)-8-((*R*)-3-amino-piperidin-1-yl)-xanthin,
(7) 1-(2-{2-[(Cyclopropylcarbonyl)amino]-phenyl}-2-oxo-ethyl)-3-methyl-7-(2-butin-1-yl)-8-((S)-3-amino-piperidin-1-yl)-xanthin und
(8) 1-[2-(2-{[(Pyridin-2-yl)carbonyl]amino}-phenyl)-2-oxo-ethyl]-3-methyl-7-((E)-2-buten-1-yl)-8-(3-amino-piperidin-1-yl)-xanthin
sowie deren Tautomere, Enantiomere, Diastereomere, deren Gemische und deren Salze.

Erfindungsgemäß erhält man die Verbindungen der allgemeinen Formel I nach an sich bekannten Verfahren, beispielsweise nach folgenden Verfahren:
a) Zur Herstellung von Verbindungen der allgemeinen Formel I, in der R⁴ einer der eingangs erwähnten, über ein Stickstoffatom mit dem Xanthingerüst verknüpften Reste ist:
   Umsetzung einer Verbindung der allgemeinen Formel 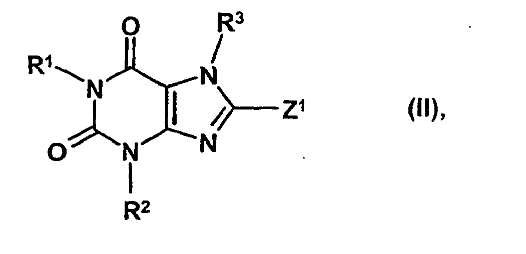 in der
   R¹ bis R³ wie eingangs erwähnt definiert sind und
   Z¹ eine Austrittsgruppe wie ein Halogenatom, eine substituierte Hydroxy-, Mercapto-, Sulfinyl-, Sulfonyl- oder Sulfonyloxygruppe, wie beispielsweise ein Chlor- oder Bromatom, eine Methansulfonyl- oder Methansulfonyloxygruppe darstellt, mit einem Amin der allgemeinen Formel R⁴'-H, in der R⁴' einen der für R⁴ eingangs erwähnten Reste darstellt, der über ein Stickstoffatom mit dem Xanthingerüst verknüpft ist.
   Die Umsetzung wird zweckmäßigerweise in einem Lösungsmittel wie Isopropanol, Butanol, Tetrahydrofuran, Dioxan, Dimethylformamid, Dimethylsulfoxid, Ethylenglycolmonomethylether, Ethylenglycoldiethylether oder Sulfolan gegebenenfalls in Gegenwart einer anorganischen oder tertiären organischen Base, z.B. Natriumcarbonat, Kaliumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladiumbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel oder in einem Überschuß des Amins der allgemeinen Formel R^{4'}-H durchgeführt werden.
b) Entschützung einer Verbindung der allgemeinen Formel 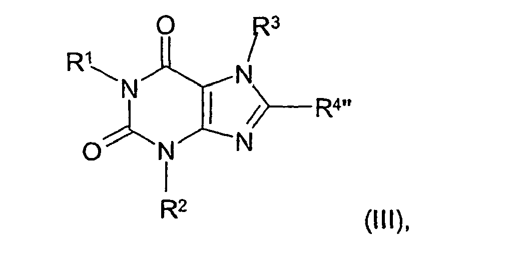 in der R¹, R² und R³ wie eingangs erwähnt definiert sind und
R⁴' eine der eingangs für R⁴ erwähnten Gruppen bedeutet, die eine Imino-, Amino- oder Alkylaminogruppe enthalten, wobei die Imino-, Amino- bzw. Alkylaminogruppe durch eine Schutzgruppe substituiert ist, gegebenenfalls gefolgt von einer nachträglichen Alkylierung der Imino-, Amino- bzw. C₁₋₃-Alkylaminogruppe.

Die Freisetzung einer Aminogruppe aus einer geschützten Vorstufe ist eine Standardreaktion in der synthetischen organischen Chemie. Als Schutzgruppen kommen eine Vielzahl von Gruppen in Frage. Eine Übersicht über die Chemie der Schutzgruppen findet sich in Theodora W.Greene und Peter G.M.Wuts, Protective Groups in Organic Synthesis, Second Edition, 1991, Verlag John Wiley and Sons sowie in Philip J. Kocienski, Protecting Groups, Georg Thieme Verlag, 1994.

Als Beispiele für Schutzgruppen seien genannt:
die tert.-Butyloxycarbonylgruppe, die sich durch Behandeln mit einer Säure wie beispielsweise Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C abspalten lässt,
die 2.2.2-Trichlorethoxycarbonylgruppe, die sich abspalten lässt durch Behandeln mit Metallen wie beispielsweise Zink oder Cadmium in einem Lösungsmittel wie Essigsäure oder einem Gemisch aus Tetrahydrofuran und einer schwachen wässrigen Säure bei Temperaturen zwischen 0°C und der Siedetemperatur des verwendeten Lösungsmittels und
die Carbobenzyloxycarbonylgruppe, die sich beispielsweise abspalten lässt durch Hydrogenolyse in Gegenwart eines Edelmetallkatalysators wie beispielsweise Palladium-Kohle und einem Lösungsmittel wie beispielsweise Alkohole, Essigester, Dioxan, Tetrahydrofuran oder Gemische dieser Lösungsmittel bei Temperaturen zwischen 0°C und dem Siedepunkt des Lösungsmittels, durch Behandeln mit Bortribromid in Methylenchlorid bei Temperaturen zwischen -20°C und Raumtemperatur, oder durch Behandeln mit Aluminiumchlorid/Anisol bei Temperaturen zwischen 0°C und Raumtemperatur.

Die gegebenenfalls nachträgliche Einführung eines C₁₋₃-Alkylrests kann mittels Alkylierung oder reduktiver-Alkylierung erfolgen.

Die nachträgliche Alkylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem Alkylierungsmittel wie einem entsprechenden Halogenid oder Sulfonsäureester, z.B. mit Methyliodid, Ethylbromid, Dimethylsulfat, gegebenenfalls in Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, durchgeführt.

Die nachträgliche reduktive Alkylierung wird mit einer entsprechenden Carbonylverbindung wie Formaldehyd, Acetaldehyd, Propionaldehyd, Aceton in Gegenwart eines komplexen Metallhydrids wie Natriumborhydrid, Lithiumborhydrid, Natriumtriacetoxyborhydrid oder Natriumcyanoborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung kann auch in Gegenwart von Ameisensäure als Reduktionsmittel bei erhöhten Temperaturen, z.B. bei Temperaturen zwischen 60 und 120°C, durchgeführt werden.

Bei den vorstehend beschriebenen Umsetzungen können gegebenenfalls vorhandene reaktive Gruppen wie Carboxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzreste für eine Carboxygruppe die Trimethylsilyl-, Methyl-, Ethyl-, tert.-Butyl-, Benzyl- oder Tetrahydropyranylgruppe,
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/VVasser oder Dioxan bei Temperaturen zwischen 20 und 50°C.

Femer können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel 1, welche in Racematen auftreten, nach an sich bekannten Methoden (siehe Allinger N. L. und Eliel E. L. in "Topics in Stereochemistry", Vol. 6, Wiley Interscience, 1971 ) in ihre optischen Antipoden und Verbindungen der allgemeinen Formel 1 mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bemsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln 11 und III sind entweder literaturbekannt oder man erhält diese nach an sich literaturbekannten Verfahren (siehe Beispiele I bis VII).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der . humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2", erschienen in Proc. Natl. Acad. Sci. Vol. 90, Seiten 5757-5761 (1993), durchgeführt. Der Zellextrakt wurde von in einem Puffer (10mM Tris HCl, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfemung von Zelltrümmern) gewonnen.

Der DPP-IV Assay wurde wie folgt durchgeführt:

50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung | DPP IV-Hemmung |
|---|---|
| (Beispiel Nr.) | IC₅₀ [nM] |
| 1 | 5 |
| 1(1) | 11 |
| 1(2) | 3 |
| 1(3) | 4 |
| 1(4 ) | 3 |
| 1(5) | 3 |
| 1(6) | 5 |
| 1(7) | 8 |

Die erfindungsgemäß hergestellten Verbindungen sind gut verträglich, da beispielsweise nach oraler Gabe von 10 mg/kg der Verbindung des Beispiels 1 (5) an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, daß sie bei jeglicher Art von Verletzung oder Beeinträchtigung im Gastrointestinaltrakt eingesetzt werden können wie auch z.B. bei Kolitiden und Enteriden. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow'scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neur degenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), alpha-Glucosidasehemmer (z.B. Acarbose, Voglibose),andere DPPIV Inhibitoren, alpha2-Antagonisten, Insulin und Insulinanaloga, GLP-1 und GLP-1 Analoga (z.B. Exendin-4) oder Amylin. Daneben SGLT2-Inhibitoren wie T-1095, Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha agonisten, PPAR-delta agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Forrnel 1, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 1-[2-(2-Formylamino-phenyl}-2-oxo-ethyl]- 3-methyl-7-(3-methyl-2-buten-1-yl)-8-[3-(tert.-butyloxycarbonylamino)-piperidin-1-yl]-xanthin

Ein Gemisch aus 1.2 ml Ameisensäure und 2 ml Essigsäureanhydrid wird für 10 Minuten auf 60°C erhitzt. Dann wird 1 ml dieser Mischung zu 226 mg 1-[2-(2-Aminophenyl)-2-oxo-ethyl]-3-methyl-7-(3-methyl-2-buten-1-yl)-8-[3-(tert.-butyloxycarbonyl-amino)-piperidin-1-yl]-xanthin gegeben und das Reaktionsgemisch wird 15 Minuten bei 80°C gerührt. Zur Aufarbeitung wird das Reaktionsgemisch mit Methylenchlorid versetzt und langsam mit gesättigter Kaliumcarbonat-Lösung alkalisch gestellt. Die wässrige Phase wird mit Methylenchlorid extrahiert und die vereinigten organischen Phasen werden über Natriumsulfat getrocknet und eingeengt. Das Rohprodukt wird ohne weitere Reinigung weiter umgesetzt.
Ausbeute: 186 mg (78 % der Theorie)
R_{f}-Wert: 0.40 (Kieselgel, Cyclohexan/Essigester = 3:7)
Massenspektrum (ESI⁺): m/z = 594 [M+H]⁺

Analog Beispiel I werden folgende Verbindungen erhalten:
(1) 1-[2-(2-Formylamino-phenyl)-2-oxo-ethyl]-3-methyl-7-(2-butin-1-yl)-8-[3-(tert.-butyloxycarbonylamino)-piperidin-1-yl]-xanthin
   R_{f}-Wert: 0.23 (Kieselgel, Cyclohexan/Essigester = 3:7)
   Massenspektrum (ESI⁺): m/z = 578 [M+H]⁺

### Beispiel II

### 1-[2-(2-Amino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Behandeln von 1-[2-(2-Nitro-phenyl)2-oxo-ethyl]-3-methyl-7-(3-methyl-2-buten-1-yl)-8-[3-(tert.-butyloxycarbonylamino)-piperidin-1-yl]-xanthin mit Eisenpulver in einem Gemisch aus Ethanol, Wasser und Eisessig (150:50:14) bei 90°C.
Massenspektrum (ESI⁺): m/z =566 [M+H]⁺

Analog Beispiel II werden folgende Verbindungen erhalten:
(1) 1-[2-(2-Amino-phenyl)-2-oxo-ethyl]-3-methyl-7-(2-butin-1-yl)-8-brom-xanthin Massenspektrum (ESI⁺): m/z = 430, 432 [M+H]⁺
(2) 1-[2-(2-Amino-phenyl)-2-oxo-ethyl]-3-methyl-7-((E)-2-buten-1-yl)-8-[(*R*)-3-(tert.-butyloxycarbonylamino)-piperidin-1-yl]-xanthin Massenspektrum (ESI⁺): m/z = 552 [M+H]⁺
(3) 1-[2-(2-Amino-phenyl)-2-oxo-ethyl]-3-methyl-7-((E)-2-buten-1-yl)-8-brom-xanthin
   R_{f}-Wert: 0.62 (Kieselgel, Cyclohexan/Essigester = 4:6)
   Massenspektrum (ESl⁺): m/z = 432, 434 [M+H]⁺

### Beispiel III

### 1-[2-(2-Nitro-phenyl)-2-oxo-ethyl]-3-methyl-7-(3-methyl-2-buten-1-yl)-8-[3-(tert.--butyloxycarbonylamino)-piperidin-1-yl]-xanthin

Zu 4.40 g 1-[2-(2-Nitro-phenyl)-2-oxo-ethyl]-3-methyl-7-(3-methyl-2-buten-1-yl)-8-chlor-xanthin und 1.30 g Natriumcarbonat in 50 ml Dimethylsulfoxid werden bei 65°C 2.20 g 3-tert.-Butyloxycarbonylamino-piperidin gegeben. Das Reaktionsgemisch wird ca. 16 h bei 65°C gerührt. Nach Abkühlung auf Raumtemperatur wird es auf ein Gemisch aus 600 ml Wasser und 100 g Eis gegossen. Der entstandene Niederschlag wird abgesaugt und mit Wasser nachgewaschen. Der Filterkuchen wird in Diethylether gelöst, die Lösung getrocknet und eingeengt. Der braune, harzige Kolbenrückstand wird mit Diisopropylether zur Kristallisation gebracht.
Ausbeute: 3.30 g (54 % der Theorie)
R_{f}-Wert: 0.52 (Kieselgel, Cyclohexan/Essigester = 3:7)
Massenspektrum (ESI⁺): m/z = 596 [M+H]⁺

Analog Beispiel III werden folgende Verbindungen erhalten:
(1) 1-[2-(2-Amino-phenyl)-2-oxo-ethyl]-3-methyl-7-(2-butin-1-yl)-8-[3-(tert.-butyloxycarbonylamino)-piperidin-1-yl]-xanthin
   R_{f}-Wert: 0.50 (Kieselgel, Methylenchlorid/Methanol = 95:5)
   Massenspektrum (ESI⁺): m/z = 550 [M+H]⁺
(2) 1-[2-(2-Nitro-phenyl)-2-oxo-ethyl]-3-methyl-7-((E)-2-buten-1-yl)-8-[(*R*)-3-(tert.-butyloxycarbonylamino)-piperidin-1-yl]-xanthin R_{f}-Wert: 0.50 (Kieselgel, Cyclohexan/Essigester = 1:2)
(3) 1-[2-(2-Amino-phenyl)-2-oxo-ethyl]-3-methyl-7-((E)-2-buten-1-yl)-8-[(*S*)-3-(tert.-butyloxycarbonylamino)-piperidin-1-yl]-xanthin
   R_{f}-Wert: 0.40 (Kieselgel, Cyclohexan/Essigester = 4:6)
   Massenspektrum (ESI⁺): m/z = 552 [M+H]⁺
(4) 3-Methyl-7-(2-butin-1-yl)-8-[(*R*)-3-(tert-butyloxycarbonylamino)-piperidin-1-yl]-xanthin
   Schmelzpunkt: 197-200°C
   Massenspektrum (ESI⁺): m/z = 417 [M+H]⁺
(5) 3-Methyl-7-(2-butin-1-yl)-8-[(*S*)-3-(tert.-butyloxycarbonylamino)-piperidin-1-yl]-xanthin
   R_{f}-Wert: 0.52 (Kieselgel, Essigester)
   Massenspektrum (ESI⁺): m/z = 417 [M+H]⁺
(6) 1-[2-(2-Amino-phenyl)-2-oxo-ethyl]-3-methyl-7-((E)-2-buten-1-yl)-8-[3-(tert.-butyloxycarbonylamino)-piperidin-1-yl]-xanthin
   R_{f}-Wert: 0.20 (Kieselgel, Cyclohexan/Essigester = 1:1)
   Massenspektrum (ESI⁺): m/z = 552 [M+H]⁺

### Beispiel IV

### 1-[2-(2-Nitro-phenyl)-2-oxo-ethyl]-3-methyl-7-(3-methyl-2-buten-1-yl)-8-chlor-xanthin

Ein Gemisch aus 6.02 g 3-Methyl-7-(3-methyl-2-buten-1-yl)-8-chlor-xanthin, 5.86 g 2-Brom-1-(2-nitro-phenyl)-ethanon und 5.00 g Kaliumcarbonat in 150 ml N,N-Dimethylformamid wird ca. 26 h bei 60°C gerührt. Zur Aufarbeitung wird das abgekühlte Reaktionsgemisch auf ein Gemisch aus 500 ml 1 N Natronlauge und 200 g Eis gegossen. Der entstandene Niederschlag wird abgesaugt und getrocknet.
Ausbeute: 6.32 g (65 % der Theorie)
R_{f}-Wert: 0.50 (Kieselgel, Cyclohexan/Essigester = 4:6)
Massenspektrum (ESI⁺): m/z = 432, 434 [M+H]⁺

Analog Beispiel IV werden folgende Verbindungen erhalten:
(1 ) 1-[2-(2-Nitro-phenyl)-2-oxo-ethyl]-3-methyl-7-(2-butin-1-yl)-8-brom-xanthin
   R_{f}-Wert: 0.77 (Kieselgel, Methylenchlorid/Methanol = 95:5)
   Massenspektrum (ESI⁺): m/z = 460, 462 [M+H]⁺
(2) 1-[2-(2-Nitro-phenyl)-2-oxo-ethyl]-3-methyl-7-((E)-2-buten-1-yl)-8-brom-xanthin
   R_{f}-Wert: 0.50 (Kieselgel, Cyclohexan/Essigester = 1:1)
   Massenspektrum (ESI⁺): m/z = 462, 464 [M+H]⁺
(3) 1-[2-(2-Nitro-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Methylenchlorid/Methanol = 95:5)
(4) 1-[2-(2-Nitro-phenyl)-2-oxo-ethyl]-3-methyl-7-(2-butin-1-yl)-8-[(*S*)-3-(tert.-butyloxycarbonylamino)-piperidin-1-yl]-xanthin
   R_{f}-Wert: 0.60 (Kieselgel, Methylenchlorid/Methanol = 95:5)
   Massenspektrum (ESI⁺): m/z = 580 [M+H]⁺

### Beispiel V

### 3-Methyl-7-(3-methyl-2-buten-1-yl)-8-chlor-xanthin

Zu 10.56 g 3-Methyl-8-chlor-xanthin und 17 ml Hünigbase in 100 ml N,N-Dimethylformamid werden 5.87 ml 1-Brom-3-methyl-2-buten gegeben. Das Reaktionsgemisch wird ca. 10 Minuten bei Raumtemperatur nachgerührt und anschließend mit 800 ml Wasser versetzt. Der entstandene helle Niederschlag wird abgesaugt, mit Ethanol und Diethylether nachgewaschen und getrocknet.
Ausbeute: 10.56 g (81 % der Theorie)
Massenspektrum (ESI⁺): m/z = 269, 271 [M+H]⁺

Analog Beispiel V werden folgende Verbindungen erhalten:
(1) 3-Methyl-7-(2-butin-1-yl)-8-brom-xanthin
   R_{f}-Wert: 0.72 (Kieselgel, Essigester)
   Massenspektrum (ESI⁺): m/z = 297, 299 [M+H]⁺
(2) 3-Methyl-7-((E)-2-buten-1-yl)-8-brom-xanthin Massenspektrum (ESI⁺): m/z = 299, 301 [M+H]⁺

### Beispiel VI

### 1-(2-{2-[(Cyclopropylcarbonyl)amino]-phenyl}-2-oxo-ethyl)-3-methyl-7-(3-methyl-2-buten-1-yl)-8-8-[3-(tert.-butyloxycarbonylamino)-piperidin-1-yl]-xanthin

Ein Gemisch aus 242 mg 1-[2-(2-Amino-phenyl)-2-oxo-ethyl]-3-methyl-7-(3-methyl-2-buten-1-yl)-8-[3-(tert.-butyloxycarbonylamino)-piperidin-1-yl]-xanthin und 44 µl Pyridin in N,N-Dimethylformamid wird mit 39 µl Cyclopropancarbonsäurechlorid versetzt und 2 h bei 80°C gerührt. Dann werden nochmals 20 µl Pyridin und 30 µl Cyclopropancarbonsäurechlorid zugegeben. Nach weiteren 10 h bei 80°C wird das abgekühlte Reaktionsgemisch mit Methylenchlorid verdünnt und mit Wasser versetzt. Die wässrige Phase wird mit Methylenchlorid extrahiert und die vereinigten organischen Phasen werden eingeengt. Das Rohprodukt wir über eine Kieselgel-Säule mit Cyclohexan/Essigester (7:3 auf 4:6) als Laufmittel gereinigt.
Ausbeute: 90 mg (33 % der Theorie)
R_{f}-Wert: 0.60 (Kieselgel, Cyclohexan/Essigester = 3:7)

Analog Beispiel Vl werden folgende Verbindungen erhalten:
(1) 1-(2-{2-[(Cyclopropylcarbonyl)amino]-phenyl}-2-oxo-ethyl)-3-methyl-7-((E}-2-buten-1-yl)-8-[(*R*)-3-(tert.-butyloxycarbonylamino)-piperidin-1-yl]-xanthin
   R_{f}-Wert: 0.30 (Kieselgel, Cyclohexan/Essigester/lsopropanol = 8:1:1)
   Massenspektrum (ESI⁺): m/z = 620 [M+H]⁺
(2) 1-(2-{2-[(Cyclopropylcarbonyl)amino]-phenyl}-2-oxo-ethyl)-3-methyl-7-((E)-2-buten-1-yl)-8-[(*S*)-3-(tert.-butyloxycarbonylamino)-piperidin-1-yl]-xanthin
   R_{f}-Wert: 0.53 (Kieselgel, Cyclohexan/Essigester/Isopropanol = 14:3:3)
   Massenspektrum (ESI⁺): m/z = 620 [M+H]⁺
(3) 1-(2-{2-[(Cyclopropylcarbonyl)amino]-phenyl}-2-oxo-ethyl)-3-methyl-7-(2-butin-1-yl)-8-[(*R*)-3-(tert.-butyloxycarbonylamino)-piperidin-1-yl]-xanthin
   R_{f}-Wert: 0.35 (Kieselgel, Methylenchlorid/Methanol = 95:5)
   Massenspektrum (ESI⁺): m/z = 618 [M+H]⁺
(4) 1-(2-{2-[(Cyclopropylcarbonyl)amino]-phenyl}-2-oxo-ethyl)-3-methyl-7-(2-butin-1-yl)-8-[(S)-3-(tert.-butyloxycarbonylamino)-piperidin-1-yl]-xanthin
   R_{f}-Wert: 0.35 (Kieselgel, Methylenchlorid/Methanol = 95:5)
   Massenspektrum (ESI⁺): m/z = 618 [M+H]⁺
(5) 1-[2-(2-{[(Pyridin-2-yl)carbonyl]amino}-phenyl)-2-oxo-ethyl]-3-methyl-7-((E)-2-buten-1-yl)-8-[3-(tert.-butyloxycarbonylamino)-piperidin-1-yl]-xanthin
   R_{f}-Wert: 0.55 (Kieselgel, Cyclohexan/Essigester/lsopropanol = 14:3:3)
   Massenspektrum (ESI⁺): m/z = 657 [M+H]⁺

### Beispiel VII

### 1-[2-(2-Amino-phenyl)-2-oxo-ethyl)-3-methyl-7-(2-butin-1-yl)-8-[(R)-3-(tert.-butyloxycarbonylamino)-piperidin-1-yl]-xanthin

hergestellt durch Reduktion von 1-[2-(2-Nitro-phenyl)-2-oxo-ethyl]-3-methyl-7-(2-butin-1-yl)-8-[(*R*)-3-(tert.-butyloxycarbonylamino)-piperidin-1-yl]-xanthin mit Natriumdithionit in einem Gemisch aus Methylglykol und Wasser (3:2) bei 100°C.
R_{f}-Wert: 0.50 (Kieselgel, Cyclohexan/Essigester = 4:6)

Analog Beispiel VII werden folgende Verbindungen erhalten:
(1) 1-[2-(2-Amino-phenyl)-2-oxo-ethyl]-3-methyl-7-(2-butin-1-yl)-8-[(S)-3-(tert.-butyloxycarbonylamino)-piperidin-1 -yl]-xanthin
   R_{f}-Wert: 0.34 (Kieselgel, Methylenchlorid/Methanol = 95:5)

### Herstellung der Endverbindungen:

### Beispiel 1

### 1-[2-(2-Formylamino-phenyl)-2-oxo-ethyl]-3-methyl-7-(3-methyl-2-buten-1-yl)-8-(3-amino-piperidin-1-yl)-xanthin

Eine Lösung aus 180 mg 1-[2-(2-Formylamino-phenyl)-2-oxo-ethyl]- 3-methyl-7-(3-methyl-2-buten-1-yl)-8-[3-(tert.-butyloxycarbonylamino )-piperidin-1-yl]-xanthin in 4 ml Methylenchlorid wird mit 1 ml Trifluoressigsäure versetzt und eine halbe Stunde bei Raumtemperatur gerührt. Zur Aufarbeitung wird das Reaktionsgemisch mit 1 N Natronlauge leicht alkalisch gestellt und die wässrige Phase wird mit Methylenchlorid extrahiert. Die vereinigten organischen Phasen werden eingeengt und über eine Kieselgel-Säule gereinigt.
Ausbeute: 130 mg (87 % der Theorie)
R_{f}-Wert: 0.38 (Reversed Phase DC-Fertigplatte (E. Merck), Acetonitril/Wasser/ Trifluoressigsäure = 100:100:0.1)
Massenspektrum (ESI⁺): m/z = 494 [M+H]⁺

Analog Beispiel 1 werden folgende Verbindungen erhalten:
(1) 1-(2-{2-[(Cyclopropylcarbonyl)amino]-phenyl}-2-oxo-ethyl)3-methyl-7-(3-methyl-2-buten-1-yl)-8-(3-amino-piperidin-1-yl)-xanthin
   R_{f}-Wert: 0.35 (Reversed Phase DC-Fertigplatte (E. Merck), Acetonitril/Wasser/ Trifluoressigsäure = 100:100:0.1)
   Massenspektrum (ESI⁺): m/z = 534 [M+H]⁺
(2) 1-[2-(2-Formylamino-phenyl)-2-oxo-ethyl]-3-methyl-7-(2-butin-1-yl)-8-(3-amino-piperidin-1-yl)-xanthin
   R_{f}-Wert: 0.20 (Kieselgel, Methylenchlorid/Methanol/konz. wässriges Ammoniak = 90:10:1)
   Massenspektrum (ESI⁺): m/z = 478 [M+H]⁺
(3) 1-(2-{2-[(Cyclopropylcarbonyl)amino]-phenyl}-2-oxo-ethyl)-3-methyl-7-((E)-2-buten-1-yl)-8-((*R*)-3-amino-piperidin-1-yl)-xanthin
   R_{f}-Wert: 0.50 (Reversed Phase DC-Fertigplatte (E. Merck), Acetonitril/Wasser/ Trifluoressigsäure = 50:50:0.1)
   Massenspektrum (ESI⁺): m/z = 520 [M+H]⁺
(4) 1-(2-{2-[(Cyclopropylcarbonyl)amino]-phenyl}-2-oxo-ethyl)-3-methyl-7-((E)-2-buten-1-yl)-8-((*S*)-3-amino-piperidin-1-yl)-xanthin
   R_{f}-Wert: 0.50 (Reversed Phase DC-Fertigplatte (E. Merck), Acetonitril/Wasser/Trifluoressigsäure = 50:50:0.1)
   Massenspektrum (ESI⁺): m/z = 520 [M+H]⁺
(5) 1-(2-{2-[(Cyclopropylcarbonyl)amino]-phenyl}-2-oxo-ethyl)-3-methyl-7-(2-butin-1-yl)-8-((*R*)-3-amino-piperidin-1-yl)-xanthin
   Massenspektrum (ESI⁺): m/z = 518 [M+H]⁺
(6) 1-(2-{2-[(Cyclopropylcarbonyl)amino]-phenyl}-2-oxo-ethyl)-3-methyl-7-(2-butin-1-y))-8-((*S*)-3-amino-piperidin-1-yl)-xanthin
   R_{f}-Wert: 0.14 (Kieselgel, Methylenchlorid/Methanol/konz. wässriges Ammoniak = 90:10:1)
   Massenspektrum (ESI⁺): m/z = 518 [M+H]⁺
(7) 1-[2-(2-{[(Pyridin-2-yl)carbonyl]amino}-phenyl)-2-oxo-ethyl]-3-methyl-7-((E)-2-buten-1-yl)-8-(3-amino-piperidin-1-yl)-xanthin
   Massenspektrum (ESI⁺): m/z = 557 [M+H]⁺

Analog den vorstehenden Beispielen und anderen literaturbekannten Verfahren können auch die folgenden Verbindungen erhalten werden:
(1) 1-(2-{2-[(Cyclobutylcarbonyl)amino]-phenyl}2-oxo-ethyl)-3-methyl-7-(3-methyl-2-buten-1-yl)-8-(3-amino-piperidin-1-yl)-xanthin
(2) 1-(2-{2-[(Cyclopentylcarbonyl)amino]-phenyl}-2-oxo-ethyl)-3-methyl-7-(3-methyl-2-buten-1-yl)-8-(3-amino-piperidin-1-yl)-xanthin
(3) 1-(2-{2-[(Cyclohexylcarbonyl)amino]-phenyl}-2-oxo-ethyl)-3-methyl-7-((E)-2-buten-1-yl)-8-(3-amino-piperidin-1-yl)-xanthin
(4) 1-(2-{2-[(Cycloheptylcarbonyl)amino]-phenyl}-2-oxo-ethyl)-3-methyl-7-(2-butin-1-yl)-8-(3-amino-piperidin-1-yl)-xanthin
(5) 1-[2-(2-{[(Bicyclo[2.2.1]heptan-1-yl)carbonyl]amino}-phenyl)-2-oxo-ethyl]-3-methyl-7-(3-methyl-2-buten-1-yl)-8-(3-amino-piperidin-1-yl)-xanthin
(6) 1-[2-(2-{[(Bicyclo[2.2.2]octan-1-yl)carbonyl)amino}-phenyl)-2-oxo-ethyl]-3-methyl-7-((E)-2-buten-1-yl)-8-(3-amino-piperidin-1-yl)-xanthin
(7) 1-[2-(2-{[(1-Cyclobuten-1-yl)carbonyl]amino}-phenyl)-2-oxo-ethyl]-3-methyl-7-(3-methyl-2-buten-1-yl)-8-(3-amino-piperidin-1-yl)-xanthin
(8) 1-[2-(2-{[(1-Cyclopenten-1-yl)carbonyl]amino}-phenyl)-2-oxo-ethyl]-3-methyl-7-[(1-cyclopenten-1-yl)methyl]-8-(3-amino-piperidin-1-yl)-xanthin
(9) 1-[2-(2-{[(1-Cyclohexen-1-yl)carbonyl]amino}-phenyl)-2-oxo-ethyl]-3-methyl-7-((E)-2-buten-1-yl)-8-(3-amino-piperidin-1-yl)-xanthin
(10) 1-[2-(2-{[(2-Oxo-cyclohexan-1-yl)carbonyl)amino}-phenyl)-2-oxo-ethyl]-3-methyl-7-[(1-cyclopenten-1-yl)methyl]-8-(3-amino-piperidin-1-yl)-xanthin
(11) 1-[2-(2-{[(2,6-Dioxo-cyclohexan-1-yl)carbonyl]amino}-phenyl)-2-oxo-ethyl]-3-methyl-7-[(1-cyclopenten-1-yl)methyl]-8-(3-amino-piperidin-1-yl)-xanthin
(12) 1-[2-(2-{[(Tetrahydro-furan-2-yl)carbonyl]amino}-phenyl)-2-oxo-ethyl]-3-methyl-7-(3-methyl-2-buten-1-yl)-8-(3-amino-piperidin-1-yl)-xanthin
(13) 1-[2-(2-{[(Tetrahydro-furan-3-yl)carbonyl]amino}-phenyl)2-oxo-ethyl]-3-methyl-7-(2-butin-1-yl)-8-(3-amino-piperidin-1-yl)-xanth in
(14) 1-[2-(2-{[(Tetrahydro-thiophen-2-yl)carbonyl]amino}-phenyl)-2-oxo-ethyl]-3-methyl-7-[(1-cyclopenten-1-yl)methyl]-8-(3-amino-piperidin-1 -yl)-xanthin
(15) 1-[2-(2-{[(Tetrahydro-thiophen-3-yl)carbonyl]amino}-phenyl)-2-oxo-ethyl]-3-methyl-7-(2-butin-1-yl)-8-(3-amino-piperidin-1-yl)-xanthin
(16) 1-[2-(2{[(1-Oxo-tetrahydro-thiophen-2-yl)carbonyl]amino}phenyl)-2-oxo-ethyl]-3-methyl-7-[(1-cyclopenten-1-yl)methyl]-8-(3-amino-piperidin-1-yl)-xanthin
(17) 1-[2-(2{[(1,1-Dioxo-tetrahydro-thiophen-2-yl)carbonyl]amino}phenyl)-2-oxo-ethyl]-3-rriethyl-7-[(1-cyclopenten-1-yl)methyl]-8-(3-amino-piperidin-1-yl)-xanthin
(18) 1-[2-(2-{[(Pyrrolidin-2-yl)carbonyl]amino}-phenyl)-2-oxo-ethyl]-3-methyl-7-(2-butin-1-yl)-8-(3-amino-piperidin-1-yl)-xanthin
(19) 1-[2-(2-{[(Pyrrolidin-3-yl)carbonyl]amino}-phenyl)-2-oxo-ethyl]-3-methyl-7-((E)-2-buten-1-yl)-8-(3-amino-piperidin-1-yl)-xanthin
(20) 1-[2-(2-{[(Tetrahydro-pyran-2-yl)carbonyl]amino}-phenyl)-2-oxo-ethyl]-3-methyl-7-(3-methyl-2-buten-1-yl)-8-(3-amino-piperidin-1-yl)-xanthin
(21) 1-[2-(2-{[([1,3]Dioxolan-4-yl)carbonyl]amino}-phenyl)-2-oxo-ethyl]-3-methyl-7-((E)-1-buten-1-yl)-8-(3-amino-piperidin-1-yl)-xanthin
(22) 1-[2-(2-{[([1,4]Dioxan-2-yl)carbonyl]amino)-phenyl)-2-oxo-ethyl]-3-methyl-7-(3-methyl-2-buten-1-yl)-8-(3-amino-piperidin-1-yl)-xanthin
(23) 1-[2-(2-{[(Morpholin-2-yl)carbonyl]amino}-phenyl)-2-oxo-ethyl]-3-methyl-7-((E)-2-buten-1-yl)-8-(3-amino-piperidin-1-yl)-xanthin
(24) 1-[2-(2-{[(Piperazin-2-yl)carbonyl]amino}-phenyl)-2-oxo-ethyl]-3-methyl-7-(3-methyl-2-buten-1-yl)-8-(3-amino-piperidin-1-yl)-xanthin
(25) 1-[2-(2-{[(5-Oxo-pyrrolidin-2-yl)carbonyl]amino}-pheny)-2-oxo-ethyl]-3-methyl-7-[(1-cyclopenten-1-yl)methyl]-8-(3-amino-piperidin-1-yl)-xanthin
(26) 1-[2-(2-{[(6-Oxo-piperidin-2-yl)carbonyl]amino}-phenyl)-2-oxo-ethyl]-3-methyl-7-(3-methyl-2-buten-1-yl)-8-(3-amino-piperidin-1-yl)-xanthin
(27) 1-[2-(2-{[(2-Oxo-oxazolidin-4-yl)carbonyl]amino}-phenyl)-2-oxo-ethyl]-3-methyl-7-((E)-1-buten-1-yl)-8-(3-amino-piperidin-1-yl)-xanthin
(28) 1-[2-(2-{[(Cyclopropylmethyl)carbonyl]amino}phenyl)-2-oxo-ethyl]-3-methyl-7-(2-butin-1-yl)-8-(3-amino-piperidin-1-yl)-xanthin
(29) 1-[2-(2-{[(Pyridin-3-yl)carbonyl]amino}-phenyl)-2-oxo-ethyl]-3-methyl-7-(2-butin-1-yl)-8-(3-amino-piperidin-1 -yl)-xanthin
(30) 1-(2-{2-[(Cyclopropylsulfonyl)amino]-pheny)}-2-oxo-ethyl)-3-methyl-7-(2-butin-1-yl)-8-(3-amino-piperidin-1-yl)-xanthin
(31) 1-(2-{2-[(Phenylsulfonyl)amino]-phenyl}-2-oxo-ethyl)-3-methyl-7-(2-butin-1-yl)-8-(3-amino-piperidin-1-yl)-xanthin
(32)1-(2-{2-[(Benzylsulfonyl)amino]-phenyl}-2-oxo-ethyl)-3-methyl-7-(2-butin-1-yl)-8-(3-amino-piperidin-1-yl)-xanthin

### Beispiel 2

### Dragées mit 75 mg Wirksubstanz

### 1 Drageekem enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Drageekerne werden mit einem Film überzogen, der im weser lichen aus Hydroxypropylmethylcellulose besteht. Die fertigen Filmdragees werden mit Bienenwachs geglänzt.
Dragéegewicht: 245 mg.

### Beispiel 3

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 4

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1.0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3.0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 6

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

Zusammensetzung:

| | |
|---|---|
| Wirkstoff | 10,0 mg |
| 0,01 n Salzsäure s.q. | |
| Aqua bidest | ad 2,0 ml |

### Herstellung.

Die Wirksubstanz wird in der erforderlichen Menge 0,01 n HCI gelöst, mit Kochsalz isotonisch gestellt, sterilfiltriert und in 2 ml Ampullen abgefüllt.

### Beispiel 9

### Ampullen mit 50 mg Wirksubstanz

Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 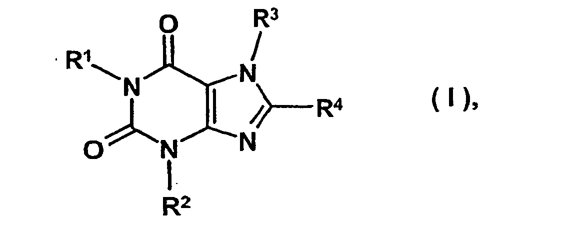 in der
R¹ eine Phenylcarbonylmethylgruppe, in der der Phenylteil durch R¹⁰ und R¹¹ substituiert ist, wobei
R¹⁰ eine Formylaminogruppe,
eine C₃₋₇-Cycloalkyl-carbonylamino- oder C₃₋₇-Cycloalkyl-C₁₋₃-alkyl-carbonylamino-Gruppe,
eine C₆₋₉-Bicycloalkyl-carbonylamino- oder C₆₋₉-Bicycloalkyl-C₁₋₃-alkyl-carbonylaminogruppe,
eine C₅₋₇-Cycloalkyl-carbonylaminogruppe, in der
eine Methylengruppe durch ein Sauerstoff- oder Schwefelatom oder durch eine Imino-, Sulfinyl- oder Sulfonylgruppe ersetzt ist,
eine C₅₋₇-Cycloalkyl-carbonylaminogruppe, in der eine -CH₂-CH₂- Gruppe durch eine -NH-CO- oder -NH-NH- Gruppe ersetzt ist,
eine C₅₋₇-Cycloalkyl-carbonylaminogruppe, in der eine -CH₂-CH₂-CH₂- Gruppe durch eine -NH-CO-NH-, -NH-CO-O- oder -O-CH₂-O- Gruppe ersetzt ist,
eine C₆₋₇-Cycloalkyl-carbonylaminogruppe, in der eine -CH₂CH₂-CH₂-CH₂₋Gruppe durch eine -NH-CH₂-CH₂-NH-, -NH-CO-CH₂-NH-, -NH-CH₂-CH₂-O-, -NH-CO-CH₂-O- oder -O-CH₂-CH₂-O-Gruppe ersetzt ist,
eine Cycloheptyl-carbonylaminogruppe, in der eine -CH₂-CH₂-CH₂-CH₂-CH₂₋Gruppe durch eine -NH-CH₂-CH₂-CH₂-NH-, -NH-CO-CH₂-CH₂-NH-, -NH-CH₂-CH₂-CH₂-O-, -NH-CO-CH₂-CH₂-O- oder -O-CH₂-CH₂-CH₂-O-Gruppe ersetzt ist,
eine C₅₋₇-Cycloalkyl-carbonylaminogruppe, in der eine oder zwei Methylengruppen durch Carbonylgruppen ersetzt sind,
eine C₄₋₇-Cycloalkenyl-carbonylamino- oder C₄₋₇-Cycloalkenyl-C₁₋₃-alkyl-carbonylamino-Gruppe,
eine C₃₋₇-Cycloalkyl-sulfonylamino-, C₃₋₇-Cycloalkyl-C₁₋₃-alkyl-sulfonylamino-, Arylsulfonylamino- oder Aryl-C₁₋₃-alkyl-sulfonylamino-Gruppe oder
eine Heteroarylcarbonylaminogruppe bedeutet,
wobei die in den vorstehend erwähnten Gruppen enthaltenen Iminogruppen unabhängig voneinander durch eine C₁₋₃-Alkylgruppe substituiert sein können,
und R¹¹ ein Wasserstoff-, Fluor-, Chlor-, Brom- oder lodatom oder
eine C₁₋₃-Alkyl-, C₁₋₃-Alkyloxy-, Difluormethyl-, Trifluorthyl-, Difluormethoxy-, Trifluormethoxy- oder Cyangruppe bedeutet,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Arylgruppe,
eine Aryl-C₁₋₄-alkylgruppe,
eine Aryl-C₂₋₃-alkenylgruppe,
eine Arylcarbonyl-C₁₋₂-alkylgruppe,
eine Heteroaryl-C₁₋₃-alkylgruppe,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-C₁₋₂-alkyl-Gruppe,
eine C₃₋₆Cycloalkyl-carbonyl-C₁₋₂-alkyl-Gruppe,
eine Aryl-D-C₁₋₃-alkylgruppe, wobei D eine Sauerstoff- oder Schwefelatom, eine Imino-, C₁₋₃-Alkylimino-, Sulfinyl- oder Sulfonylgruppe bedeutet,
eine durch eine Gruppe Rₐ substituierte C₁₋₄-Alkylgruppe, wobei
Rₐ eine Cyano-, Carboxy-, C₁₋₃-Alkyloxy-carbonyl-, Aminocarbonyl-, C₁₋₃₋Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet,
oder eine durch eine Gruppe R_{b} substituierte C₂₋₄-Alkylgruppe, wobei
R_{b} eine Hydroxy-, C₁₋₃-Alkyloxy-, Amino-, C₁₋₃-Alkylamino-, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1 -yl-Gruppe darstellt und durch mindestens zwei Kohlenstoffatome vom Ringstickstoffatom in 3-Stellung des Xanthingerüstes isoliert ist,
R³ eine C₃₋₈-Alkylgruppe,
eine durch eine Gruppe R_{c} substituierte C₁₋₃-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇₋Cycloalkenylgruppe,
eine Arylgruppe oder
eine Furanyl-, Thienyl-, Oxazolyl-, Isoxazolyl-, Thiazolyl-, lsothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom, oder eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen -substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃₋alkyl)-aminogruppe substituiert ist,
eine C₃₋₇-Cycloalkylgruppe, die durch eine Amino-C₁₋₃-alkyl-, C₁₋₃-Alkylamino-C₁₋₃₋alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃₋alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃₋Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃₋alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃₋alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃₋alkylgruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino-Teils getrennt ist, wobei R¹⁹ wie vorstehend erwähnt definiert ist,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃₋Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinotinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxochinazolinyl-, 1,2-Dihydro-2-oxochinoxalinyl-, 1,2,3,4-Tetrahydro-2,3-dioxochinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2*H*-benzo[1,4]oxazinyl-Gruppe zu verstehen ist,
und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische, deren Prodrugs und deren Salze.

2. Verbindungen der allgemeinen Formel I gemäß Anspruch 1, in denen
R¹, R² und R³ wie in Anspruch 1 erwähnt definiert sind und
R⁴ eine Pyrrolidin-1-ylgruppe, die in 3-Stellung durch eine Aminogruppe substituiert ist,
eine Piperidin-1-ylgruppe, die in 3-Stellung durch eine Aminogruppe substituiert ist,
eine Hexahydroazepin-1-yl-gruppe, die in 3-Stellung oder in 4-Stellung durch eine Aminogruppe substituiert ist,
eine (2-Aminocyclohexyl)amino-Gruppe,
eine Cyclohexylgruppe, die in 3-Stellung durch eine Aminogruppe substituiert ist, oder
eine N-(2-Aminoethyl)-methylamino- oder eine N-(2-Aminoethyl)-ethylamino-Gruppe bedeutet,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische und Salze.

3. Verbindungen der allgemeinen Formel I gemäß Anspruch 2, in denen
R¹ eine Phenylcarbonylmethylgruppe, in der der Phenylteil durch R¹⁰ substituiert ist, wobei
R¹⁰ eine Formylaminogruppe,
eine C₃₋₇-Cycloalkyl-carbonylamino- oder C₃₋₇-Cycloalkyl-C₁₋₃-alkyl-carbonylamino-Gruppe,
eine C₆₋₉-Bicycloalkyl-carbonylaminogruppe,
eine C₅₋₇-Cycloalkyl-carbonylaminogruppe, in der
eine Methylengruppe durch ein Sauerstoff- oder Schwefelatom oder durch eine Imino-, Sulfinyl- oder Sulfonylgruppe ersetzt ist,
eine (1,3-Dioxoianyl)-carbonylamino-, (1,4-Dioxanyl)-carbonylamino-, Morpholin-2-yl-carbonylamino-, Morpholin-3-ylcarbonylamino- oder Piperazin-2-yl-carbonylamino-Gruppe,
eine C₅₋₇-Cycloalkyl-carbonylaminogruppe, in der eine -CH₂-CH₂- Gruppe durch eine -NH-CO- Gruppe ersetzt ist,
eine C₅₋₇-Cycloalkyl-carbonylaminogruppe, in der eine -CH₂-CH₂-CH₂- Gruppe durch eine -NH-CO-O- Gruppe ersetzt ist,
eine C₅₋₇-Cycloalkyl-carbonylaminogruppe, in der eine Methylengruppe durch eine Carbonylgruppe ersetzt ist,
eine C₅₋₇-Cycloalkenyl-carbonylamino- oder C₅₋₇-Cycloalkenyl-C₁₋₃-alkyl-carbonylamino-Gruppe, .
eine C₃₋₇-Cycloalkyl-sulfonylamino-, Phenylsulfonylamino- oder Phenyl-C₁₋₃₋alkyl-sulfonylamino-Gruppe oder
eine Pyridinylcarbonylaminogruppe bedeutet,
R² ein Wasserstoffatom,
oder eine C₁₋₃-Alkylgruppe,
R³ eine C₄₋₆-Alkenylgruppe,
eine 2-Butin-1-ylgruppe oder
eine 1-Cyclopenten-1-y1-methy4-Gruppe
und
R⁴ eine Piperidin-1-ylgruppe, die in 3-Stellung durch eine Aminogruppe substituiert ist,
eine Hexahydroazepin-1-yl-gruppe, die in 3-Stellung oder in 4-Stellung durch eine Aminogruppe substituiert ist,
eine (2-Aminocyclohexyl)amino-Gruppe,
eine Cyclohexylgruppe, die in 3-Stellung durch eine Aminogruppe substituiert ist, oder
eine N-(2-Aminoethyl)-methylamino- oder eine N-(2-Aminoethyl)-ethylamino-Gruppe bedeuten,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische und deren Salze.

4. Verbindungen der allgemeinen Formel I gemäß Anspruch 3, in denen
R¹ eine Phenylcarbonylmethyl-Gruppe, in der der Phenylteil durch eine Formylamino, Pyridinylcarbonylamino- oder Cyclopropylcarbonylamino-Gruppe substituiert ist,
R² eine Methylgruppe,
R³ eine 2-Buten-1-yl- oder 3-Methyl-2-buten-1-yl-Gruppe oder
eine 2-Butin-1-yl-Gruppe
und
R⁴ eine (3-Amino-piperidin-1-yl)-Gruppe bedeuten,
deren Tautomere, Enantiomere, Diastereomere, deren Gemische und deren Salze.

5. Verbindungen der allgemeinen Formel I gemäß Anspruch 4, in denen
R¹ eine [2-(Cyclopropylcarbonylamino)-phenyl]-carbonylmethyl-oder [2-(Pyridylcarbonylamino)-phenyl]-carbonylmethyl-Gruppe,
R² eine Methylgruppe,
R³ eine 2-Buten-1-yl- oder 3-Methyl-2-buten-1-yl-Gruppe oder
eine 2-Butin-1-yl-Gruppe
und
R⁴ eine (3-Amino-piperidin-1-yl)-Gruppe bedeuten,
deren Tautomere, Enantiomere, Diastereomere, deren Gemische und deren Salze.

6. Folgenden Verbindungen der allgemeinen Formel I gemäß Anspruch 1:
(1) 1-[2-(2-Formylamino-phenyl)-2-oxo-ethyl]-3-methyl-7-(3-methyl-2-buten-1-yl)-8-(3-amino-piperidin-1-yl)-xanthin,
(2) 1-(2-{2-[(Cyclopropylcarbonyl)amino]-phenyl}-2-oxo-ethyl)-3-methyl-7-(3-methyl-2-buten-1-yl)-8-(3-amino-piperidin-1-yl)-xanthin,
(3) 1-[2-(2-Formylamino-phenyl)-2-oxo-ethyl]-3-methyl-7-(2-butin-1-yl)-8-(3-amino-piperidin-1-yl)-xanthin,
(4) 1-(2-{2-[(Cyclopropylcarbonyl)amino]-phenyl}-2-oxo-ethyl)-3-methyl-7-((E)-2-buten-1-yl)-8-((*R*)-3-amino-piperidin-1-yl)-xanthin,
(5) 1-(2-{2-[(Cyclopropylcarbonyl)amino]-phenyl}-2-oxo-ethyl)-3-methyl-7-((E)-2-buten-1-yl)-8-((*S*)-3-amino-piperidin-1-yl)-xanthin,
(6) 1-(2-{2-[(Cyclopropylcarbonyl)amino]-phenyl}-2-oxo-ethyl)-3-methyl-7-(2-butin-1-yl)-8-((*R*)-3-amino-piperidin-1-yl)-xanthin,
(7) 1-(2-{2-[(Cyclopropylcarbonyl)amino]-phenyl)-2-oxo-ethyl)-3-methyl-7-(2-butin-1-yl)-8-((*S*)-3-amino-piperidin-1-yl)-xanthin und
(8) 1-[2-(2-{[(Pyridin-2-yl)carbonyl]amino}-phenyl)-2-oxo-ethyl]-3-methyl-7-((E)-2-buten-1-yl)-8-(3-amino-piperidin-1-yl)-xanthin
sowie deren Tautomere, Enantiomere, Diastereomere, deren Gemische und deren Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

10. Verfahren zur Herstellung eines Arzneimittels gemäß Anspruch 8, **dadurch gekennzeichnet, daß** auf nichtchemischen Weg eine Verbindung nach mindestens einem der Ansprüche 1 bis 7 in einen oder mehrere inerte Trägerstoffe und/oder Verdünnungsmittel eingearbeitet wird.

11. Verfahren zur Herstellung der Verbindungen der allgemeinen Formel 1 gemäß den Ansprüchen 1 bis 7, **dadurch gekennzeichnet, daß**
a) zur Herstellung von Verbindungen der allgemeinen Formel I, in der R⁴ einer der in Anspruch 1 erwähnten, über ein Stickstoffatom mit dem Xanthingerüst verknüpften Reste ist
eine Verbindung der allgemeinen Formel 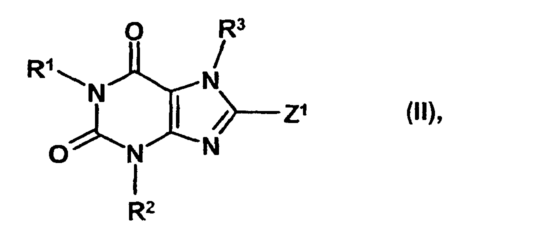 in der
R¹ bis R³ wie in Anspruch 1 erwähnt definiert sind und
Z¹ eine Austrittsgruppe wie ein Halogenatom, eine substituierte Hydroxy-, Mercapto-, Sulfinyl-, Sulfonyl- oder Sulfonyloxygruppe darstellt,
mit einem Amin der allgemeinen Formel R⁴'-H, in der R⁴' einen der für R⁴ in Anspruch 1 erwähnten Reste darstellt, der über ein Stickstoffatom mit dem Xanthingerüst verknüpft ist, umgesetzt wird, oder
b) eine Verbindung der allgemeinen Formel 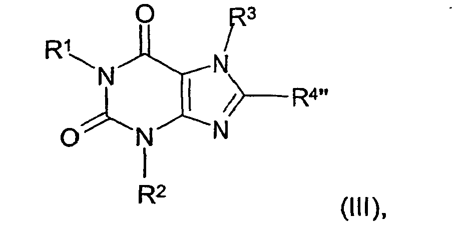
in der R¹, R² und R³ wie in Anspruch 1 erwähnt definiert sind und
R⁴' eine der eingangs für R⁴ erwähnten Gruppen bedeutet, die eine Imino-, Amino- oder Alkylaminogruppe enthalten, wobei die Imino-, Amino- bzw. Alkylaminogruppe durch eine Schutzgruppe substituiert ist, entschützt und anschließend an der Imino-, Amino- bzw. C₁₋₃-Alkylaminogruppe gegebenenfalls alkyliert wird, und/oder
anschließend gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel 1 in ihre Salze, insbesondere für die pharmazeutische Anwendung in ihre physiologisch verträglichen Salze mit anorganischen oder organischen Säuren oder Basen, übergeführt werden.

## Claims

1. Compounds of general formula 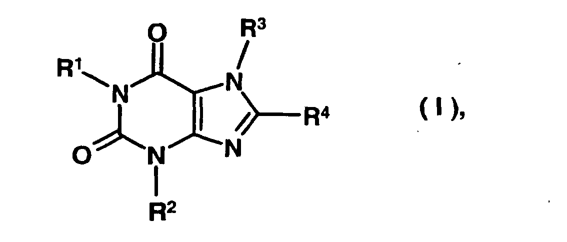 wherein
R¹ denotes a phenylcarbonylmethyl group wherein the phenyl moiety is substituted by R¹⁰ and R¹¹, where
R¹⁰ denotes a formylamino group,
a C₃-₇-cycloalkyl-carbonylamino or C₃₋₇-cycloalkyl-C₁₋₃-alkyl-carbonylamino group,
a C₆₋₉-bicycloalkyl-carbonylamino or C₆₋₉-bicycloalkyl-C₁₋₃-alkylcarbonylamino group,
a C₅₋₇-cycloalkyl-carbonylamino group wherein
a methylene group is replaced by an oxygen or sulphur atom or by an imino, sulphinyl or sulphonyl group,
a C₅₋₇-cycloalkyl-carbonylamino group wherein a -CH₂-CH₂ group is replaced by a -NH-CO or -NH-NH group,
a C₅₋₇-cycloalkyl-carbonylamino group wherein a -CH₂-CH₂-CH₂ group is replaced by a -NH-CO-NH, -NH-CO-O or -O-CH₂-O group,
a C₆₋₇-cycloalkyl-carbonylamino group wherein a -CH₂-CH₂-CH₂-CH₂ group is replaced by a -NH-CH₂-CH₂-NH, -NH-CO-CH₂-NH, -NH-CH₂₋CH₂-O, -NH-CO-CH₂-O or -O-CH₂-CH₂-O group,
a cycloheptyl-carbonylamino group wherein a -CH₂-CH₂-CH₂-CH₂-CH₂ group is replaced by a -NH-CH₂-CH₂-CH₂-NH, -NH-CO-CH₂-CH₂-NH, -NH-CH₂-CH₂-CH₂-O, -NH-CO-CH₂-CH₂-O or -O-CH₂-CH₂-CH₂-O group,
a C₅₋₇-cycloalkyl-carbonylamino group wherein one or two methylene groups are replaced by carbonyl groups,
a C₄₋₇-cycloalkenyl-carbonylamino or C₄₋₇-cycloalkenyl-C₁₋₃-alkylcarbonylamino group,
a C₃₋₇-cycloalkyl-sulphonylamino, C₃₋₇-cycloalkyl-C₁₋₃-alkylsulphonylamino, arylsulphonylamino or aryl-C₁₋₃-alkyl-sulphonylamino group or
a heteroarylcarbonylamino group,
while the imino groups contained in the above mentioned groups may be substituted independently of one another by a C₁₋₃-alkyl group,
and R¹¹ denotes a hydrogen, fluorine, chlorine, bromine or iodine atom or
a C₁₋₃-alkyl, C₁₋₃-alkyloxy, difluoromethyl, trifluoromethyl,
difluoromethoxy, trifluoromethoxy or cyano group,
R² denotes a hydrogen atom,
a C₁₋₆-alkyl group,
a C₂₋₄-alkenyl group,
a C₃₋₄-alkynyl group,
a C₃₋₆-cycloalkyl group,
a C₃₋₆-cycloalkyl-C₁₋₃-alkyl group,
a tetrahydrofuran-3-yl, tetrahydropyran-3-yl, tetrahydropyran-4-yl, tetrahydrofuranylmethyl or tetrahydropyranylmethyl group,
an aryl group,
an aryl-C₁₋₄-alkyl group,
an aryl-C₂₋₃-alkenyl group,
an arylcarbonyl-C₁₋₂-alkyl group,
a heteroaryl-C₁₋₃-alkyl group,
a furanylcarbonylmethyl, thienylcarbonylmethyl, thiazolylcarbonylmethyl or pyridylcarbonylmethyl group,
a C₁₋₄-alkyl-carbonyl-C₁₋₂-alkyl group,
a C₃₋₆-cycloalkyl-carbonyl-C₁₋₂-alkyl group,
an aryl-D-C₁₋₃-alkyl group, while D denotes an oxygen or sulphur atom, an imino, C₁₋₃-alkylimino, sulphinyl or sulphonyl group,
a C₁₋₄-alkyl group substituted by a group Rₐ, where
Rₐ denotes a cyano, carboxy, C₁₋₃-alkyloxy-carbonyl, aminocarbonyl, C₁₋₃-alkylamino-carbonyl, di-(C₁₋₃-alkyl)-amino-carbonyl, pyrrolidin-1-ylcarbonyl, piperidin-1-ylcarbonyl, morpholin-4-ylcarbonyl, piperazin-1-ylcarbonyl, 4-methylpiperazin-1-ylcarbonyl or 4-ethylpiperazin-1-ylcarbonyl group,
or a C₂₋₄-alkyl group substituted by a group R_{b}, where
R_{b} denotes a hydroxy, C₁₋₃-alkyloxy, amino, C₁₋₃-alkylamino, di-(C₁₋₃₋alkyl)-amino, pyrrolidin-1-yl, piperidin-1-yl, morpholin-4-yl, piperazin-1-yl, 4-methyl-piperazin-1-yl or 4-ethyl-piperazin-1-yl group and is isolated from the cyclic nitrogen atom in the 3 position of the xanthine skeleton by at least two carbon atoms,
R³ denotes a C₃₋₈-alkyl group,
a C₁₋₃-alkyl group substituted by a group R_{c}, where
R_{c} denotes a C₃₋₇-cycloalkyl group optionally substituted by one or two C₁₋₃-alkyl groups,
a C₅₋₇-cycloalkenyl group optionally substituted by one or two C₁₋₃-alkyl groups,
an aryl group or
a furanyl, thienyl, oxazolyl, isoxazolyl, thiazolyl, isothiazolyl-, pyridyl, pyridazinyl, pyrimidyl or pyrazinyl group, while the above mentioned heterocyclic groups may each be substituted by one or two C₁₋₃-alkyl groups or by a fluorine, chlorine, bromine or iodine atom or by a trifluoromethyl, cyano or C₁₋₃-alkyloxy group,
a C₃₋₈-alkenyl group,
a C₃₋₆-alkenyl group substituted by a fluorine, chlorine or bromine atom, or a trifluoromethyl group,
a C₃₋₈-alkynyl group,
an aryl group or
an aryl-C₂₋₄-alkenyl group,
and
R⁴ denotes an azetidin-1-yl or pyrrolidin-1-yl group which is substituted in the 3 position by an amino, C₁₋₃-alkylamino or a di-(C₁₋₃-alkyl)amino group and may additionally be substituted by one or two C₁₋₃-alkyl groups,
a piperidin-1-yl or hexahydroazepin-1-yl group which is substituted in the 3 position or 4 position by an amino, C₁₋₃-alkylamino or a di-(C₁₋₃-alkyl)amino group and may additionally be substituted by one or two C₁₋₃-alkyl groups,
a 3-amino-piperidin-1-yl group wherein the piperidin-1-yl moiety is additionally substituted by an aminocarbonyl, C₁₋₂-alkyl-aminocarbonyl, di-(C₁₋₂₋alkyl)aminocarbonyl, pyrrolidin-1-yl-carbonyl, (2-cyano-pyrrolidin-1-yl)-carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety is additionally substituted in the 4 position or 5 position by a hydroxy or methoxy group,
a 3-amino-piperidin-1-yl group wherein the methylene group in the 2 position or 6 position is replaced by a carbonyl group,
a piperidin-1-yl or hexahydroazepin-1-yl group substituted in the 3 position by an amino, C₁₋₃-alkylamino or di-(C₁₋₃-alkyl)-amino group, wherein in each case two hydrogen atoms on the carbon skeleton of the piperidin-1-yl or hexahydroazepin-1-yl group are replaced by a straight-chain alkylene bridge, this bridge containing 2 to 5 carbon atoms if the two hydrogen atoms are on the same carbon atom, or 1 to 4 carbon atoms if the hydrogen atoms are on adjacent carbon atoms, or 1 to 4 carbon atoms if the hydrogen atoms are on carbon atoms which are separated by one atom, or 1 to 3 carbon atoms if the hydrogen atoms are on carbon atoms which are separated by two atoms,
an azetidin-1-yl, pyrrolidin-1-yl, piperidin-1-yl or hexahydroazepin-1-yl group which is substituted by an amino-C₁₋₃-alkyl, C₁₋₃-alkylamino-C₁₋₃-alkyl or a di-(C₁₋₃-alkyl)amino-C₁₋₃-alkyl group,
a piperazin-1-yl or [1,4]diazepan-1-yl group optionally substituted on the carbon skeleton by one or two C₁₋₃-alkyl groups,
a 3-imino-piperazin-1-yl, 3-imino-[1,4]diazepan-1-yl or 5-imino-[1,4]diazepan-1-yl group optionally substituted on the carbon skeleton by one or two C₁₋₃₋alkyl groups,
a [1,4]diazepan-1-yl group optionally substituted by one or two C₁₋₃-alkyl groups which is substituted in the 6 position by an amino group,
a C₃₋₇-cycloalkyl group which is substituted by an amino, C₁₋₃-alkylamino or di-(C₁₋₃-alkyl)-amino group,
a C₃₋₇-cycloalkyl group which is substituted by an amino-C₁₋₃-alkyl, C₁₋₃₋alkylamino-C₁₋₃-alkyl or a di-(C₁₋₃-alkyl)amino-C₁₋₃-alkyl group,
a C₃₋₇-cycloalkyl-C₁₋₂-alkyl group wherein the cycloalkyl moiety is substituted by an amino, C₁₋₃-alkylamino or di-(C₁₋₃-alkyl)-amino group,
a C₃₋₇-cycloalkyl-C₁₋₂-alkyl group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃₋alkyl group,
a C₃₋₇-cycloalkylamino group wherein the cycloalkyl moiety is substituted by an amino, C₁₋₃-alkylamino or di-(C₁₋₃-alkyl)-amino group, while the two nitrogen atoms on the cycloalkyl moiety are separated from one another by at least two carbon atoms,
an N-(C₃₋₇-cycloalkyl)-N-(C₁₋₃-alkyl)-amino group wherein the cycloalkyl moiety is substituted by an amino, C₁₋₃-alkylamino or di-(C₁₋₃-alkyl)-amino group, while the two nitrogen atoms on the cycloalkyl moiety are separated from one another by at least two carbon atoms,
a C₃₋₇-cycloalkylamino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃₋alkyl group,
an N-(C₃₋₇-cycloalkyl)-N-(C₁₋₃-alkyl)-amino group wherein the cycloalkyl moiety , is substituted by an amino-C₁₋₃-alkyl, C₁₋₃-alkylamino-C₁₋₃-alkyl or a di-(C₁₋₃₋alkyl)amino-C₁₋₃-alkyl group,
a C₃₋₇-cycloalkyl-C₁₋₂-alkyl-amino group wherein the cycloalkyl moiety is substituted by an amino, C₁₋₃-alkylamino or di-(C₁₋₃-alkyl)-amino group,
an N-(C₃₋₇-cycloalkyl-C₁₋₂-alkyl)-N-(C₁₋₂-alkyl)-amino group wherein the cycloalkyl moiety is substituted by an amino, C₁₋₃-alkylamino or di-(C₁₋₃-alkyl)-amino group,
a C₃₋₇-cycloalkyl-C₁₋₂-alkyl-amino group wherein the cycloalkyl moiety is substituted by an amino-C₁₋₃-alkyl, C₁₋₃-alkylamino-C₁₋₃-alkyl or a di-(C₁₋₃₋alkyl)amino-C₁₋₃-alkyl group,
an N-(C₃₋₇-cycloalkyl-C₁₋₂-alkyl)-N-(C₁₋₂-alkyl)-amino group wherein the cycloalkyl moiety is substituted by an amino-C₁₋₃-alkyl, C₁₋₃-alkylamino-C₁₋₃₋alkyl or a di-(C₁₋₃-alkyl)amino-C₁₋₃-alkyl group,
an R¹⁹-C₂₋₄-alkylamino group wherein R¹⁹ is separated from the nitrogen atom of the C₂₋₄-alkylamino moiety by at least two carbon atoms and
R¹⁹ denotes an amino, C₁₋₃-alkylamino or di-(C₁₋₃-alkyl)-amino group,
an R¹⁹-C₂₋₄-alkylamino group wherein the nitrogen atom of the C₂₋₄-alkylamino moiety is substituted by a C₁₋₃-alkyl group and R¹⁹ is separated from the nitrogen atom of the C₂₋₄-alkylamino moiety by at least two carbon atoms, where R¹⁹ is as hereinbefore defined,
an amino group substituted by the group R²⁰ wherein
R²⁰ denotes an azetidin-3-yl, azetidin-2-ylmethyl, azetidin-3-ylmethyl, pyrrolidin-3-yl, pyrrolidin-2-ylmethyl, pyrrolidin-3-ylmethyl, piperidin-3-yl, piperidin-4-yl, piperidin-2-ylmethyl, piperidin-3-ylmethyl or piperidin-4-ylmethyl group, while the groups mentioned for R²⁰ may each be substituted by one or two C₁₋₃-alkyl groups,
an amino group substituted by the group R²⁰ and a C₁₋₃-alkyl group wherein R²⁰ is as hereinbefore defined, while the groups mentioned for R²⁰ may each be substituted by one or two C₁₋₃-alkyl groups,
an R¹⁹-C₃₋₄-alkyl group wherein the C₃₋₄-alkyl moiety is straight-chained) and may additionally be substituted by one or two C₁₋₃-alkyl groups, where R¹⁹ is as hereinbefore defined,
a 3-amino-2-oxo-piperidin-5-yl or 3-amino-2-oxo-1-methyl-piperidin-5-yl group,
a pyrrolidin-3-yl; piperidin-3-yl, piperidin-4-yl, hexahydroazepin-3-yl or hexahydroazepin-4-yl group which is substituted in the 1 position by an amino, C₁₋₃-alkylamino or di-(C₁₋₃-alkyl)amino group,
or an azetidin-2-yl-C₁₋₂-alkyl, azetidin-3-yl-C₁₋₂-alkyl, pyrrolidin-2-yl-C₁₋₂-alkyl, pyrrolidin-3-yl, pyrrolidin-3-yl-C₁₋₂-alkyl, piperidin-2-yl-C₁₋₂alkyl, piperidin-3-yl, piperidin-3-yl-C₁₋₂-alkyl, piperidin-4-yl or piperidin-4-yl-C₁₋₂-alkyl group, while the abovementioned groups may each be substituted by one or two C₁₋₃-alkyl groups,
while by the aryl groups mentioned in the definition of the above groups are meant phenyl or naphthyl groups, which may be mono- or disubstituted by Rₕ independently of one another, where the substituents are identical or different and Rₕ denotes a fluorine, chlorine, bromine or iodine atom, a trifluoromethyl, cyano, nitro, amino, aminocarbonyl, aminosulphonyl, methylsulphonyl, acetylamino, methylsulphonylamino, C₁₋₃-alkyl, cyclopropyl, ethenyl, ethynyl, hydroxy, C₁₋₃-alkyloxy, difluoromethoxy or trifluoromethoxy group,
by the heteroaryl groups mentioned in the definitions of the above mentioned groups are meant a pyrrolyl, furanyl, thienyl, pyridyl, indolyl, benzofuranyl, benzothiophenyl, quinolinyl or isoquinolinyl group,
or a pyrrolyl, furanyl, thienyl or pyridyl group wherein one or two methyne groups are replaced by nitrogen atoms,
or an indolyl, benzofuranyl, benzothiophenyl, quinolinyl or isoquinolinyl group wherein one to three methyne groups are replaced by nitrogen atoms,
or a 1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl, 1,2,3,4-tetrahydro-2,3-dioxo-pyrazinyl, 2,3-dihydro-2-oxo-indolyl, 2,3-dihydrobenzofuranyl, 2,3-dihydro-2-oxo-1 *H*-benzimidazolyl, 2,3-dihydro-2-oxo-benzoxazolyl, 1,2-dihydro-2-oxo-quinolinyl, 1,4-dihydro-4-oxo-quinolinyl, 1,2-dihydro-l-oxo-isoquinolinyl, 1,4-dihydro-4-oxo-cinnolinyl, 1,2-dihydro-2-oxo-quinazolinyl, 3,4-dihydro-4-oxo-quinazolinyl, 1,2,3,4-tetrahydro-2,4-dioxo-quinazolinyl, 1,2-dihydro-2-oxoquinoxalinyl, 1,2,3,4-tetrahydro-2,3-dioxo-quinoxalinyl, 1,2-dihydro-1-oxo-phthalazinyl, 1,2,3,4-tetrahydro-1,4-dioxo-phthalazinyl, chromanyl, cumarinyl, 2,3-dihydro-benzo[1,4]dioxinyl or 3,4-dihydro-3-oxo-2*H*-benzo[1,4]oxazinyl group,
and the above mentioned heteroaryl groups may be mono- or disubstituted by Rₕ, while the substituents may be identical or different and Rₕ is as hereinbefore defined,
and, unless otherwise stated, the above mentioned alkyl, alkenyl and alkynyl groups may be straight-chain or branched,
the tautomers, enantiomers, diastereomers, the mixtures thereof, the prodrugs thereof and the salts thereof.

2. Compounds of general formula I according to claim 1, wherein
R¹, R² and R³ are defined as in claim 1 and
R⁴ denotes a pyrrolidin-1-yl. group which is substituted in the 3 position by an amino group,
a piperidin-1-yl group which is substituted in the 3 position by an amino group,
a hexahydroazepin-1-yl group which is substituted in the 3 position or 4 position by an amino group,
a (2-aminocyclohexyl)amino group,
a cyclohexyl group which is substituted in the 3 position by an amino group, or
an N-(2-aminoethyl)-methylamino or an N-(2-aminoethyl)-ethylamino group,
while, unless otherwise stated, the above mentioned alkyl, alkenyl and alkynyl groups may be straight-chain or branched,
the tautomers, enantiomers, diastereomers, the mixtures thereof and salts thereof.

3. Compounds of general formula I according to claim 2, wherein
R¹ denotes a phenylcarbonylmethyl group wherein the phenyl moiety is substituted by R¹⁰, while
R¹⁰ denotes a formylamino group,
a C₃₋₇-cycloalkyl-carbonylamino or C₃₋₇-cycloalkyl-C₁₋₃-alkyl-carbonylamino group,
a C₆₋₉-bicycloalkyl-carbonylamino group,
a C₅₋₇-cycloalkyl-carbonylamino group wherein
a methylene group is replaced by an oxygen or sulphur atom or by an imino, sulphinyl or sulphonyl group,
a (1,3-dioxolanyl)-carbonylamino, (1,4-dioxanyl)-carbonylamino, morpholin-2-yl-carbonylamino, morpholin-3-ylcarbonylamino or piperazin-2-yl-carbonylamino group,
a C₅₋₇-cycloalkyl-carbonylamino group wherein a -CH₂-CH₂ group is replaced by an -NH-CO group,
a C₅₋₇-cycloalkyl-carbonylamino group wherein a -CH₂-CH₂-CH₂ group is replaced by an -NH-CO-O group,
a C₅₋₇-cycloalkyl-carbonylamino group wherein a methylene group is replaced by a carbonyl group,
a C₅₋₇-cycloalkenyl-carbonylamino or C₅₋₇-cycloalkenyl-C₁₋₃-alkyl-carbonylamino group,
a C₃₋₇-cycloalkyl-sulphonylamino, phenylsulphonylamino or phenyl-C₁₋₃₋alkyl-sulphonylamino group or
a pyridinylcarbonylamino group,
R² denotes a hydrogen atom,
or a C₁₋₃-alkyl group,
R³ denotes a C₄₋₆-alkenyl group,
a 2-butyn-1-yl group or
a 1-cyclopenten-1-yl-methyf group
and
R⁴ denotes a piperidin-1-yl group which is substituted in the 3 position by an amino group,
a hexahydroazepin-1-yl group which is substituted in the 3 position or 4 position by an amino group,
a (2-aminocyclohexyl)amino group,
a cyclohexyl group which is substituted in the 3 position by an amino group, or
an N-(2-aminoethyl)-methylamino or an N-(2-aminoethyl)-ethylamino group,
while, unless otherwise stated, the above mentioned alkyl, alkenyl and alkynyl groups may be straight-chain or branched,
the tautomers, enantiomers, diastereomers, the mixtures thereof and the salts thereof.

4. Compounds of general formula I according to claim 3, wherein
R¹ denotes a phenylcarbonylmethyl group wherein the phenyl moiety is substituted by a formylamino, pyridinylcarbonylamino or cyclopropylcarbonylamino group,
R² denotes a methyl group,
R³ denotes a 2-buten-1-yl or 3-methyt-2-buten-1-yl group or
a 2-butyn-1-yl group
and
R⁴ denotes a (3-amino-piperidin-1-yl) group,
the tautomers, enantiomers, diastereomers, the mixtures thereof and the salts thereof.

5. Compounds of general formula I according to claim 4, wherein
R¹ denotes a [2-(cyclopropylcarbonylamino)-phenyl]-carbonylmethyl or [2-(pyridylcarbonylamino)-phenyl]-carbonylmethyl group,
R² denotes a methyl group,
R³ denotes a 2-buten-1-yl or 3-methyl-2-buten-1-yl group or
a 2-butyn-1-yl group
and
R⁴ denotes a (3-amino-piperidin-1-yl) group,
the tautomers, enantiomers, diastereomers, the mixtures thereof and the salts thereof.

6. The following compounds of general formula according to claim 1:
(1) 1-[2-(2-formylamino-phenyl)-2-oxo-ethyl]-3-methyl-7-(3-methyl-2-buten-1-yl)-8-(3-amino-piperidin-1-yl)-xanthine,
(2) 1-(2-{2-[(cyclopropylcarbonyl)amino]-phenyl}-2-oxo-ethyl)-3-methyl-7-(3-methy)-2-buten-1-y)-8-(3-amino-piperidin-1-yl)-xanthine,
(3) 1-[2-(2-formylamino-phenyl)-2-oxo-ethyl]-3-methyl-7-(2-butyn-1-yl)-8-(3-amino-piperidin-1-yl)-xanthine,
(4) 1-(2-(2-[(cyclopropylcarbonyl)amino]-phenyl}-2-oxo-ethyl)-3-methyl-7-((E)-2-buten-1-yl)-8-((*R*)-3-amino-piperidin-1-yl)-xanthine,
(5) 1-(2-{2-[(cyclopropylcarbonyl)amino]-phenyl}-2-oxo-ethyl)-3-methyl-7-((E)-2-buten-1-yl)-8-((*S*)-3-amino-piperidin-1-yl)-xanthine,
(6) 1-(2-{2-[(cyclopropylcarbonyl)amino]-phenyl}-2-oxo-ethyl)-3-methyl-7-(2-butyn-1-yl)-8-((*R*)-3-amino-piperidin-1-yl)-xanthine,
(7) 1-(2-{2-[(cyclopropylcarbonyl)amino]-phenyl}-2-oxo-ethyl)-3-methyl-7-(2-butyn-1-yl)-8-((*S*)-3-amino-piperidin-1-yl)-xanthine and
(8) 1-[2-(2-{[(pyridin-2-yl)carbonyl]amino}-phenyl)-2-oxo-ethyl]-3-methyl-7-((E)-2-buten-1-yl)-8-(3-amino-piperidin-1-yl)-xanthine
as well as the tautomers, enantiomers, diastereomers, the mixtures thereof and the salts thereof.

7. Physiologically acceptable salts of the compounds according to at least one of claims 1 to 6 with inorganic or organic acids or bases.

8. Pharmaceutical compositions containing a compound according to at least one of claims 1 to 6 or a physiologically acceptable salt according to claim 7 optionally together with one or more inert carriers and/or diluents.

9. Use of a compound according to at least one of claims 1 to 7 for preparing a pharmaceutical composition which is suitable for treating type I and type II diabetes mellitus, arthritis, obesity, allograft transplantation and calcitonin-induced osteoporosis.

10. Process for preparing a pharmaceutical composition according to claim 8, **characterised in that** a compound according to at least one of claims 1 to 7 is incorporated in one or more inert carriers and/or diluents by a non-chemical method.

11. Process for preparing the compounds of general formula I according to claims 1 to 7, **characterised in that**
a) in order to prepare compounds of general formula I wherein R⁴ is one of the groups mentioned in claim 1 linked to the xanthine skeleton via a nitrogen atom:
a compound of general formula - 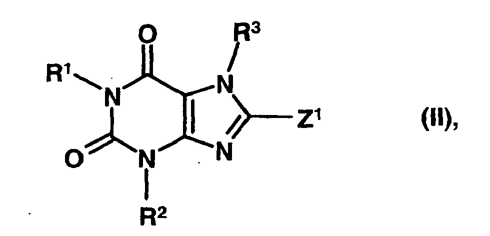 wherein
R¹ to R³ are defined as in claim 1 and
Z¹ denotes a leaving group such as a halogen atom, a substituted hydroxy, mercapto, sulphinyl, sulphonyl or sulphonyloxy group,
is reacted with an amine of general formula R⁴'-H, wherein R⁴' denotes one of the groups mentioned for R⁴ in claim 1 which is linked to the xanthine skeleton via a nitrogen atom, or
b) a compound of general formula 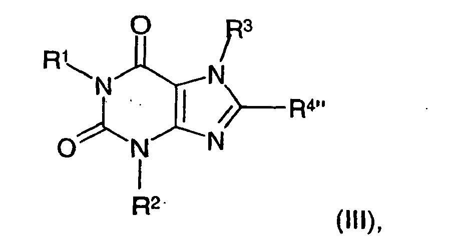
wherein R¹, R² and R³ are defined as in claim 1 and
R⁴ denotes one of the groups mentioned for R⁴ hereinbefore which contain an imino, amino or alkylamino group, while the imino, amino or alkylamino group is substituted by a protective group, is deprotected, and is subsequently optionally alkylated at the imino, amino or C₁₋₃-alkylamino group, and/or subsequently, if desired, any protecting groups used during the reaction are cleaved and/or
the compounds of general formula I thus obtained are resolved into their enantiomers or diastereomers and/or
the compounds of formula I thus obtained are converted into the salts thereof, particularly for pharmaceutical use into the physiologically acceptable salts thereof with inorganic or organic acids or bases.

## Revendications

1. Composés de formule générale 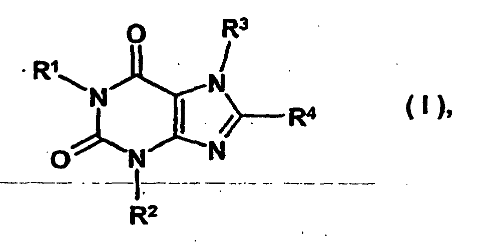 où
R¹ représente un groupe phénylcarbonylméthyle où la partie phényle est substituée par R¹⁰ et R¹¹, où
R¹⁰ représente un groupe formylamino,
un groupe C₃₋₇-cycloalkyl-carbonylamino ou C₃₋₇-cycloalkyl-C₁₋₃₋alkylcarbonylamino,
un groupe C₆₋₉-bicycloalkyl-carbonylamino ou C₆₋₉-bicycloalkyl-C₁₋₃₋alkylcarbonylamino,
un groupe C₅₋₇-cycloalkyl-carbonylamino, où
un groupe méthylène est remplacé par un atome d'oxygène ou de soufre ou par un groupe imino, sulfinyle ou sulfonyle,
un groupe C₅₋₇-cycloalkyl-carbonylamino où un groupe -CH₂-CH₂- est remplacé par un groupe -NH-CO- ou -NH-NH-,
un groupe C₅₋₇-cycloalkyl-carbonylamino où un groupe -CH₂-CH₂-CH₂- est remplacé par un groupe NH-CO-NH-, -NH-CO-O- ou -O-CH₂-O-,
un groupe C₆₋₇-cycloalkyl-carbonylamino où un groupe -CH₂-CH₂-CH₂₋CH₂- est remplacé par un groupe -NH-CH₂-CH₂-NH-, -NH-CO-CH₂-NH-,-NH-CH₂-CH₂-O-, -NH-CO-CH₂-O- ou -O-CH₂-CH₂-O-
un groupe cycloheptyl-carbonylamino où un groupe -CH₂-CH₂-CH₂-CH₂₋CH₂- est remplacé par un groupe NH-CH₂-CH₂-CH₂-NH-, -NH-CO-CH₂₋CH₂-NH-, -NH-CH₂-CH₂-CH₂-O-, -NH-CO-CH₂-CH₂-O- ou -O-CH₂-CH₂₋CH₂-O-,
un groupe C₅₋₇-cycloalkyl-carbonylamino où un ou deux groupes méthylène sont remplacés par des groupes carbonyle,
un groupe C₄₋₇-cycloalcényl-carbonylamino ou C₄₋₇-cycloalcényl-C₁₋₃-alkyl-carbonylamino,
un groupe C₃₋₇-cycloalkyl-sulfonylamino ou C₃₋₇-cycloalkyl-C₁₋₃-alkyl-sulfonylamino, arylsulfonylamino ou aryl-C₁₋₃-alkyl-sulfonylamino ou un groupe hétéroarylcarbonylamino,
où les groupes imino contenus dans les groupes cités précédemment peuvent être substitués indépendamment les uns des autres par un groupe C₁₋₃-alkyle, et R¹¹ représente un atome d'hydrogène, de fluor, de chlore, de brome ou d'iode ou
un groupe C₁₋₃-alkyle, C₁₋₃-alkyloxy, difluorométhyle, trifluorométhyle, difluorométhoxy, trifluorométhoxy ou cyano,
R² représente un atome d'hydrogène,
un groupe C₁₋₆-alkyle,
un groupe C₂₋₄-alcényle,
un groupe C₃₋₄-alcynyle,
un groupe C₃₋₆-cycloalkyle,
un groupe C₃₋₆-cycloalkyl-C₁₋₃-alkyle,
un groupe tétrahydrofuran-3-yle, tétrahydropyran-3-yle, tétrahydropyran-4-yle, tétrahydrofuranylméthyle ou tétrahydropyranylméthyle,
un groupe aryle,
un groupe aryl-C₁₋₄-alkyle,
un groupe aryl-C₂₋₃-alcényle,
un groupe arylcarbonyl-C₁₋₂-alkyle,
un groupe hétéroaryl-C₁₋₃-alkyle,
un groupe furanylcarbonylméthyle, thiénylcarbonylméthyle, thiazolylcarbonylméthyle ou pyridylcarbonylméthyle,
un groupe C₁₋₄-alkyl-carbonyl-C₁₋₂-alkyle,
un groupe C₃₋₆-cycloalkyl-carbonyl-C₁₋₂-alkyle,
un groupe aryl-D-C₁₋₃-alkyle, où D représente un atome d'oxygène ou de soufre, un groupe imino, C₁₋₃-alkylimino, sulfinyle ou sulfonyle,
un groupe C₁₋₄-alkyle substitué par un groupe Rₐ, où
Rₐ représente un groupe cyano, carboxy, C₁₋₃-alkyloxy-carbonyle, aminocarbonyle, C₁₋₃-alkylamino-carbonyle, di-(C₁₋₃-alkyl)-aminocarbonyle, pyrrolidin-1-ylcarbonyle, pipéridin-1-ylcarbonyle, morpholin-4-ylcarbonyle, pipérazin-1-ylcarbonyle, 4-méthylpipérazin-1-ylcarbonyle ou 4-éthylpipérazin-1-ylcarbonyle,
ou un groupe C₂₋₄-alkyle substitué par un groupe R_{b}, où
R_{b} représente un groupe hydroxy, C₁₋₃-alkyloxy, amino, C₁₋₃-alkylamino, di-(C₁₋₃-alkyl)-amino, pyrrolidin-1-yle, pipéridin-1-yle, morpholin-4-yle, pipérazin-1-yle, 4-méthyl-pipérazin-1-yle ou 4-éthyl-pipérazin-1-yle et est isolé de l'atome d'azote cyclique en position 3 du squelette xanthine par au moins deux atomes de carbone,
R³ représente un groupe C₃₋₈-alkyle,
un groupe C₁₋₃-alkyle substitué par un groupe R_{c}, où
R_{c} représente un goupe C₃₋₇-cycloalkyle éventuellement substitué par un ou deux groupes C₁₋₃-alkyle,
un groupe C₅₋₇-cycloalcényle éventuellement substitué par un ou deux groupes C₁₋₃-alkyle,
un groupe aryle ou
un groupe furanyle, thiényle, oxazolyle, isoxazolyle, thiazolyle, isothiazolyle, pyridyle, pyridazinyle, pyrimidyle ou pyrazinyle, où les groupements hétérocycliques cités précédemment peuvent être substitués dans chaque cas par un ou deux groupes C₁₋₃-alkyle ou par un atome de fluor, de chlore, de brome ou d'iode ou par un groupe trifluorométhyle, cyano ou C₁₋₃-alkyloxy,
un groupe C₃₋₈-alcényle,
un groupe C₃₋₆-alcényle substitué par un atome de fluor, de chlore ou de brome, ou
un groupe trifluorométhyle,
un groupe C₃₋₈-alcynyle,
un groupe aryle ou
un groupe aryl-C₂₋₄-alcényle,
et
R⁴ représente un groupe azétidin-1-yle ou pyrrolidin-1-yle, qui est substitué en position 3 par un groupe amino, C₁₋₃-alkylamino ou un groupe di-(C₁₋₃₋alkyl)amino et qui peut en outre être substitué par un ou deux groupes C₁₋₃-alkyle, un groupe pipéridin-1-yle ou hexahydroazépin-1-yle, qui est substitué en position 3 ou en position 4 par un groupe amino, C₁₋₃-alkylamino ou un groupe di-(C₁₋₃₋alkyl)amino et qui peut en outre être substitué par un ou deux groupes C₁₋₃-alkyle, un groupe 3-amino-pipéridin-1-yle, où la partie pipéridin-1-yle est substituée en outre par un groupe aminocarbonyle, C₁₋₂-alkyl-aininocarbonyle, di-(C₁₋₂₋alkyl)aminocarbonyle, pyrrolidin-1-yl-carbonyle, (2-cyanopyrrolidin-1-yl)-carbonyle, thiazolidin-3-yl-carbonyle, (4-cyanothiazolidin-3-yl)carbonyle, pipéridin-1-ylcarbonyle ou morpholin-4-ylcarbonyle,
un groupe 3-amino-pipéridin-1-yle où la partie pipéridin-1-yle est substituée en outre en position 4 ou en position 5 par un groupe hydroxy ou méthoxy,
un groupe 3-amino-pipéridin-1-yle où le groupe méthylène en position 2 ou en position 6 est remplacé par un groupe carbonyle,
un groupe pipéridin-1-yle ou hexahydroazépin-1-yle substitué en position 3 par un groupe amino, C₁₋₃-alkylamino ou di-(C₁₋₃-alkyl)-amino où dans chaque cas deux atomes d'hydrogène sur le squelette carboné du groupe pipéridin-1-yle ou hexahydroazépin-1-yle sont remplacés par un pont alkylène linéaire, où ce pont contient 2 à 5 atomes de carbone quand les deux atomes d'hydrogène se trouvent sur le même atome de carbone, ou contient 1 à 4 atomes de carbone quand les atomes d'hydrogène se trouvent sur des atomes de carbone voisins, ou contient 1 à 4 atomes de carbone quand les atomes d'hydrogène se trouvent sur des atomes de carbone qui sont séparés par un atome, ou contient 1 à 3 atomes de carbone quand les deux atomes d'hydrogène se trouvent sur des atomes de carbone qui sont séparés par deux atomes,
un groupe azétidin-1-yle, pyrrolidin-1-yle, pipéridin-1-yle ou hexahydroazépin-1-yle qui est substitué par un groupe amino-C₁₋₃-alkyle, C₁₋₃-alkylamino-C₁₋₃-alkyle ou un groupe di-(C₁₋₃-alkyl)-amino-C₁₋₃-alkyle,
un groupe pipérazin-1-yle ou [1,4]diazépan-1-yle éventuellement substitué sur le squelette carboné par un ou deux groupes C₁₋₃-alkyle,
un groupe 3-imino-pipérazin-1-yle, 3-imino-[1,4]diazépan-1-yle ou 5-imino[1,4]diazépan-1-yle éventuellement substitué sur le squelette carboné par un ou deux groupes C₁₋₃-alkyle,
un groupe [1,4]diazépan-1-yle éventuellement substitué par un ou deux groupes C₁₋₃-alkyle qui est substitué en position 6 par un groupe amino,
un groupe C₃₋₇-cycloalkyle qui est substitué par un groupe amino, C₁₋₃-alkylamino ou di-(C₁₋₃-alkyl)-amino,
un groupe C₃₋₇-cycloalkyle qui est substitué par un groupe amino-C₁₋₃-alkyle, C₁₋₃₋alkylamino-C₁₋₃-alkyle ou di-(C₁₋₃-alkyl)-amino-C₁₋₃-alkyle,
un groupe C₃₋₇-cycloalkyl-C₁₋₂-alkyle où la partie cycloalkyle est substituée par un groupe amino, C₁₋₃-alkylamino ou di-(C₁₋₃-alkyl)-amino,
un groupe C₃₋₇-cycloalkyl-C₁₋₂-alkyle où la partie cycloalkyle est substituée par un groupe amino-C₁₋₃-alkyle, C₁₋₃-alkylamino-C₁₋₃-alkyle ou un groupe di-(C₁₋₃₋alkyl)-amino-C₁₋₃-alkyle,
un groupe C₃₋₇-cycloalkylamino où la partie cycloalkyle est substituée par un groupe amino, C₁₋₃-alkylamino ou di-(C₁₋₃-alkyl)-amino, où les deux atomes d'azote sur la partie cycloalkyle sont séparés l'un de l'autre par au moins deux atomes de carbone,
un groupe N-(C₃₋₇-cycloalkyl)-N-(C₁₋₃-alkyl)-amino où la partie cycloalkyle est substituée par un groupe amino, C₁₋₃-alkylamino ou di-(C₁₋₃-alkyl)-amino, où les deux atomes d'azote sur la partie cycloalkyle sont séparés l'un de l'autre par au moins deux atomes de carbone,
un groupe C₃₋₇-cycloalkylamino où la partie cycloalkyle est substituée par un groupe amino-C₁₋₃-alkyle, C₁₋₃-alkylamino-C₁₋₃-alkyle ou di-(C₁₋₃-alkyl)-amino-C₁₋₃-alkyle,
un groupe N-(C₃₋₇-cycloalkyl)-N-(C₁₋₃-alkyl)-amino où la partie cycloalkyle est substituée par un groupe amino-C₁₋₃-alkyle, C₁₋₃-alkylamino-C₁₋₃-alkyle ou un groupe di-(C₁₋₃-alkyl)-amino-C₁₋₃-alkyle,
un groupe C₃₋₇-cycloalkyl-C₁₋₂-alkyl-amino où la partie cycloalkyle est substituée par un groupe amino, C₁₋₃-alkylamino ou di-(C₁₋₃-alkyl)-amino,
un groupe N-(C₃₋₇-cycloalkyl-C₁₋₂-alkyl)-N-(C₁₋₂-alkyl)-amino où la partie cycloalkyle est substituée par un groupe amino, C₁₋₃-alkylamino ou di-(C₁₋₃-alkyl)-amino,
un groupe C₃₋₇-cycloalkyl-C₁₋₂-alkyl-amino où la partie cycloalkyle est substituée par un groupe amino-C₁₋₃-alkyle, C₁₋₃-alkylamino-C₁₋₃-alkyle ou un groupe di-(C₁₋₃-alkyl)-amino-C₁₋₃-alkyle,
un groupe N-(C₃₋₇-cycloalkyl-C₁₋₂-alkyl)-N-(C₁₋₂-alkyl)-amino où la partie cycloalkyle est substituée par un groupe amino-C₁₋₃-alkyle, C₁₋₃-alkylamino-C₁₋₃₋alkyle ou un groupe di-(C₁₋₃-alkyl)-amino-C₁₋₃-alkyle,
un groupe R¹⁹-C₂₋₄-O-alkylamino où R¹⁹ est séparé de l'atome d'azote de la partie C₂₋₄-alkylamino par au moins deux atomes de carbone et
R¹⁹ représente un groupe amino, C₁₋₃-alkylamino ou di-(C₁₋₃-alkyl)-amino, un groupe R¹⁹-C₂₋₄-alkylamino où l'atome d'azote de la partie C₂₋₄-alkylamino est substitué par un groupe C₁₋₃-alkyle et R¹⁹ est séparé de l'atome d'azote de la partie C₂₋₄-alkylamino par au moins deux atomes de carbone, où R¹⁹ est défini comme indiqué précédemment,
un groupe amino substitué par le groupement R²⁰, où
R²⁰ représente un groupe azétidin-3-yle, azétidin-2-ylméthyle, azétidin-3-ylméthyle, pyrrolidin-3-yle, pyrrolidin-2-ylméthyle, pyrrolidin-3-ylméthyle, pipéridin-3-yle, pipéridin-4-yle, pipéridin-2-ylméthyle, pipéridin-3-ylméthyle ou pipéridin-4-ylméthyle, où les groupements cités pour R²⁰ peuvent être substitués dans chaque cas par un ou deux groupes C₁₋₃-alkyle,
un groupe amino substitué par le groupement R²⁰ et un groupe C₁₋₃-alkyle, où R²⁰ est défini comme indiqué précédemment, où les groupements cités pour R²⁰ peuvent être substitués dans chaque cas par un ou deux groupes C₁₋₃-alkyle,
un groupe R¹⁹-C₃₋₄-alkyle où la partie C₃₋₄-alkyle est linéaire et peut être substituée en outre par un ou deux groupes C₁₋₃-alkyle, où R¹⁹ est défini comme indiqué précédemment,
un groupe 3-amino-2-oxo-pipéridin-5-yle ou 3-amino-2-oxo-1-méthyl-pipéridin-5-yle,
un groupe pyrrolidin-3-yle, pipéridin-3-yle, pipéridin-4-yle, hexahydroazépin-3-yle ou hexahydroazépin-4-yle qui est substitué en position 1 par un groupe amino, C₁₋₃-alkylamino ou di-(C₁₋₃-alkyl)-amino,
ou un groupe azétidin-2-yl-C₁₋₂-alkyle, azétidin-3-yl-C₁₋₂-alkyle, pyrrolidin-2-yl-C₁₋₂-alkyle, pyrrolidin-3-yle, pyrrolidin-3-yl-C₁₋₂-alkyle, pipéridin-2-yl-C₁₋₂-alkyle, pipéridin-3-yle, pipéridin-3-yl-C₁₋₂-alkyle, pipéridin-4-yle ou pipéridin-4-yl-C₁₋₂₋alkyle, où les groupes cités précédemment peuvent être substitués dans chaque cas par un ou deux groupes C₁₋₃-alkyle,
où, par les groupes aryle cités lors de la définition des groupements cités précédemment, on doit comprendre des groupes phényle ou naphtyle qui peuvent être mono- ou disubstitués indépendamment les uns des autres par Rₕ, où les substituants peuvent être identiques ou différents et Rₕ représente un atome de fluor, de chlore, de brome ou d'iode, un groupe trifluorométhyle, cyano, nitro, amino, aminocarbonyle, aminosulfonyle, méthylsulfonyle, acétylamino, méthylsulfonylamino, C₁₋₃-alkyle, cyclopropyle, éthényle, éthynyle, hydroxy, C₁₋₃₋alkyloxy, difluorométhoxy ou trifluorométhoxy,
par les groupes hétéroaryle cités lors de la définition des groupements cités précédemment on doit comprendre un groupe pyrrolyle, furanyle, thiényle, pyridyle, indolyle, benzofuranyle, benzothiophényle, quinolinyle ou isoquinolinyle,
ou on doit comprendre un groupe pyrrolyle, furanyle, thiényle ou pyridyle où un ou deux groupes méthyne sont remplacés par des atomes d'azote,
ou on doit comprendre un groupe indolyle, benzofuranyle, benzothiophényle, quinolinyle ou isoquinolinyle où un à trois groupes méthyne sont remplacés par des atomes d'azote,
ou on doit comprendre un groupe 1,2-dihydro-2-oxo-pyridinyle, 1,4-dihydro-4-oxo-pyridinyle, 2,3-dihydro-3-oxo-pyridazinyle, 1,2,3,6-tétrahydro-3,6-dioxo-pyridazinyle, 1,2-dihydro-2-oxo-pyrimidinyle, 3,4-dihydro-4-oxo-pyrimidinyle, 1,2,3,4-tétrahydro-2,4-dioxo-pyrimidinyle, 1,2-dihydro-2-oxo-pyrazinyle, 1,2,3,4-tétrahydro-2,3-dioxo-pyrazinyle, 2,3-dihydro-2-oxo-indolyle, 2,3-dihydrobenzofuranyle, 2,3-dihydro-2-oxo-1*H*-benzimidazolyle, 2,3-dihydro-2-oxo-benzoxazolyle, 1,2-dihydro-2-oxo-quinolinyle, 1,4-dihydro-4-oxo-quinolinyle, 1,2-dihydro-1-oxo-isoquinolinyle, 1,4-dihydro-4-oxo-cinnolinyle, 1,2-dihydro-2-oxo-quinazolinyle, 3,4-dihydro-4-oxo-quinazolinyle, 1,2,3,4-tétrahydro-2,4-dioxoquinazolinyle, 1,2-dihydro-2-oxoquinoxalinyle, 1,2,3,4-tétrahydro-2,3-dioxoquinoxalinyle, 1,2-dihydro-1-oxo-phtalazinyle, 1,2,3,4-tétrahydro-1,4-dioxo-phtalazinyle, chromanyle, coumarinyle, 2,3-dihydro-benzo[1,4]dioxinyle ou 3,4-dihydro-3-oxo-2*H*-benzo[1,4]oxazinyle,
et les groupes hétéroaryle cités précédemment peuvent être mono- ou disubstitués par Rₕ, où les substituants peuvent être identiques ou différents
et Rₕ est défini comme indiqué précédemment,
où, sauf indication contraire, les groupes alkyle, alcényle et alcynyle cités précédemment peuvent être linéaires ou ramifiés,
leurs tautomères, énantiomères, diastéréoisomères, leurs mélanges, leurs promédicaments et leurs sels.

2. Composés de formule générale I selon la revendication 1 où
R¹, R² et R³ sont définis comme indiqué dans la revendication 1 et
R⁴ représente un groupe pyrrolidin-1-yle qui est substitué en position 3 par un groupe amino,
un groupe pipéridin-1-yle qui est substitué en position 3 par un groupe amino,
un groupe hexahydroazépin-1-yle qui est substitué en position 3 ou en position 4 par un groupe amino,
un groupe (2-aminocyclohexyl)amino,
un groupe cyclohexyle qui est substitué en position 3 par un groupe amino, ou
un groupe N-(2-aminoéthyl)-méthylamino ou un groupe N-(2-aminoéthyl)-éthylamino,
où, sauf indication contraire, les groupes alkyle, alcényle et alcynyle cités précédemment peuvent être linéaires ou ramifiés,
leurs tautomères, énantiomères, diastéréoisomères, leurs mélanges et leurs sels.

3. Composés de formule générale I selon la revendication 2 où
R¹ représente un groupe phénylcarbonylméthyle où la partie phényle est substituée par R¹⁰, où
R¹⁰ représente un groupe formylamino,
un groupe C₃₋₇-cycloalkyl-carbonylamino ou C₃₋₇-cycloalkyl-C₁₋₃₋alkylcarbonyl-amino,
un groupe C₆₋₉-bicycloalkyl-carbonylamino,
un groupe C₅₋₇-cycloalkyl-carbonylamino, où
un groupe méthylène est remplacé par un atome d'oxygène ou de soufre ou par un groupe imino, sulfinyle ou sulfonyle,
un groupe (1,3-dioxolanyl)-carbonylamino, (1,4-dioxanyl)-carbonylamino, morpholin-2-yl-carbonylamino, morpholin-3-ylcarbonylamino ou pipérazin-2-yl-carbonylamino,
un groupe C₅₋₇-cycloalkyl-carbonylamino où un groupe -CH₂-CH₂- est remplacé par un groupe NH-CO-,
un groupe C₅₋₇-cycloalkyl-carbonylamino où un groupe -CH₂-CH₂-CH₂- est remplacé par un groupe -NH-CO-O-,
un groupe C₅₋₇-cycloalkyl-carbonylamino où un groupe méthylène est remplacé par un groupe carbonyle,
un groupe C₅₋₇-cycloalcényl-carbonylamino ou C₅₋₇-cycloalcényl-C₁₋₃-alkyl-carbonylamino,
un groupe C₃₋₇-cycloalkyl-sulfonylamino, phénylsulfonylamino ou phényl-C₁₋₃-alkyl-sulfonylamino ou
un groupe pyridinylcarbonylamino,
R² représente un atome d'hydrogène,
un groupe C₁₋₃-alkyle,
R³ représente un groupe C₄₋₆-alcényle,
un groupe 2-butyn-1-yle ou
un groupe 1-cyclopentén- 1 -ylméthyle
et
R⁴ représente un groupe pipéridin-1-yle qui est substitué en position 3 par un groupe amino,
un groupe hexahydroazépin-1-yle qui est substitué en position 3 ou en position 4 par un groupe amino,
un groupe (2-aminocyclohexyl)amino,
un groupe cyclohexyle qui est substitué en position 3 par un groupe amino
ou
un groupe N-(2-aminoéthyl)-méthylamino ou un groupe N-(2-aminoéthyl)-éthylamino,
où, sauf indication contraire, les groupes alkyle, alcényle et alcynyle cités précédemment peuvent être linéaires ou ramifiés,
leurs tautomères, énantiomères, diastéréoisomères, leurs mélanges et leurs sels.

4. Composés de formule générale I selon la revendication 3 où
R¹ représente un groupe phénylcarbonylméthyle où la partie phényle est substituée par un groupe formylamino, pyridinylcarbonylamino ou cyclopropylcarbonylamino,
R² représente un groupe méthyle,
R³ représente un groupe 2-butén-1-yle ou 3-méthyl-2-butén-1-yle ou
un groupe 2-butyn-1-yle
et
R⁴ représenté un groupe (3-amino-pipéridin-1-yle),
leurs tautomères, énantiomères, diastéréoisomères, leurs mélanges et leurs sels.

5. Composés de formule générale I selon la revendication 4 où
R¹ représente un groupe [2-(cyclopropylcarbonylamino)-phényl]-carbonylméthyle
ou [2-(pyridylcarbonylamino)-phényl]-carbonylméthyle,
R² représente un groupe méthyle,
R³ représente un groupe 2-butén-1-yle ou 3-méthyl-2-butén-1-yle ou
un groupe 2-butyn-1-yle
et
R⁴ représente un groupe (3-amino-pipéridin-1-yle),
leurs tautomères, énantiomères, diastéréoisomères, leurs mélanges et leurs sels.

6. Composés de formule générale I selon la revendication 1 suivants :
(1) 1-[2-(2-formylamino-phényl)-2-oxo-éthyl]-3-méthyl-7-(3-méthyl-2-butén-1-yl)-8-(3-amino-pipéridin-1-yl)-xanthine,
(2) 1-(2-{2-[(cyclopropylcarbonyl)amino]-phényl}-2-oxo-éthyl)-3-méthyl-7-(3-méthyl-2-butén-1-yl)-8-(3-amino-pipéridin-1-yl)-xanthine,
(3) 1-[2-(2-formylamino-phényl)-2-oxo-éthyl]-3-méthyl-7-(2-butyn-1-yl)-8-(3-amino-pipéridin-1-yl)-xanthine,
(4) 1-(2-{2-[(cyclopropylcarbonyl)amino]-phényl}-2-oxo-éthyl)-3-méthyl-7-((E)-2-butén-1-yl)-8-((*R*)-3-amino-pipéridin-1-yl)-xanthine,
(5) 1-(2-{2-[(cyclopropylcarbonyl)amino]-phényl}-2-oxo-éthyl)-3-méthyl-7-((E)-2-butén-1-yl)-8-((*S*)-3-amino-pipéridin-1-yl)-xanthine,
(6) 1-(2-{2-[(cyclopropylcarbonyl)amino]-phényl}-2-oxo-éthyl)-3-méthyl-7-(2-butyn-1-yl)-8-((*R*)-3-amino-pipéridin-1-yl)-xanthine,
(7) 1-(2-{2-[(cyclopropylcarbonyl)amino]-phényl}-2-oxo-éthyl)-3-méthyl-7-(2-butyn-1-yl)-8-((*S*)-3-amino-pipéridin-1-yl)-xanthine et
(8) 1-[2-(2-{[(pyridin-2-yl)carbonyl]amino}-phényl)-2-oxo-éthyl]-3-méthyl-7-((E)-2-butén-1-yl)-8-(3-amino-pipéridin-1-yl)-xanthine,
ainsi que leurs tautomères, énantiomères, diastéroisomères, leurs mélanges et leurs sels.

7. Sels physiologiquement acceptables des composés selon au moins l'une des revendications 1 à 6 avec des acides ou des bases inorganiques ou organiques.

8. Médicament contenant un composé selon au moins l'une des revendications 1 à 6 ou un sel physiologiquement acceptable selon la revendication 7 outre éventuellement un ou plusieurs supports et/ou diluants inertes.

9. Utilisation d'un composé selon au moins l'une des revendications 1 à 7 pour la production d'un médicament qui convient pour le traitement du diabète sucré de type I et de type II, de l'arthrite, de l'adiposité, de la transplantation d'allogreffes et de l'ostéoporose provoquée par la calcitonine.

10. Procédé de production d'un médicament selon la revendication 8 **caractérisé en ce que**, par voie non chimique, un composé selon au moins l'une des revendications 1 à 7 est incorporé dans un ou plusieurs supports et/ou diluants inertes.

11. Procédé de production des composés de formule générale I selon les revendications 1 à 7 **caractérisé en ce que**
a) pour la production de composés de formule générale 1 où R⁴ est l'un des groupements liés au squelette xanthine par un atome d'azote cités dans la revendication 1
un composé de formule générale 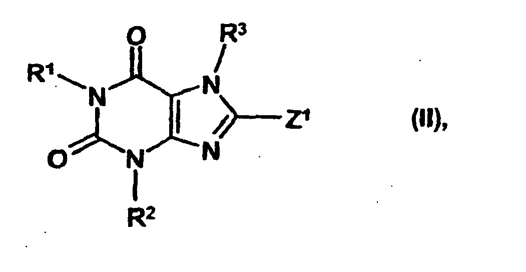 où
R¹ à R³ sont définis comme indiqué dans la revendication 1 et
Z¹ représente un groupe partant comme un atome d'halogène, un groupe hydroxy, mercapto, sulfinyle, sulfonyle ou sulfoxyloxy substitué,
est mis à réagir avec une amine de formule générale R^{4'}-H où R^{4'} représente l'un des groupements cités pour R⁴ dans la revendication 1, qui est lié au squelette xanthine par un atome d'azote, ou
b) un composé de formule générale 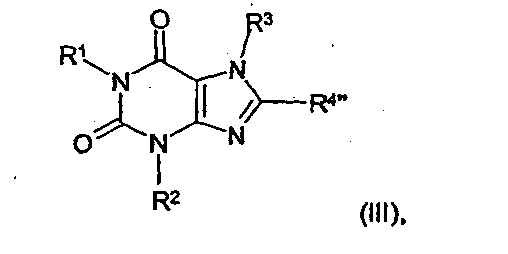
où
R¹, R² et R³ sont définis comme indiqué dans la revendication 1 et
R^{4"} représente l'un des groupements cités au début pour R⁴ qui contiennent un groupe imino, amino ou alkylamino, où le groupe imino, amino ou alkylamino est substitué par un groupe protecteur, est déprotégé puis éventuellement alkylé au niveau du groupe imino, amino ou C₁₋₃-alkylamino, et/ou
ensuite les groupes protecteurs éventuellement utilisés pendant la réaction sont clivés et/ou
les composés de formule générale 1 ainsi obtenus sont séparés en leurs énantiomères et/ou diastéréoisomères et/ou
les composés de formule générale 1 obtenus sont convertis en leurs sels, en particulier pour l'utilisation pharmaceutique. en leurs sels physiologiquement acceptables avec des acides ou des bases inorganiques ou organiques.
